# EUROPÄISCHE PATENTANMELDUNG

(11) **EP 2 597 082 A1**
(43) Veröffentlichungstag der Anmeldung: **29.05.2013**
(21) Anmeldenummer: 11190461.1
(22) Anmeldetag: 24.11.2011
(51) Int. Cl.: C07C 233/49, A23C 9/13, A23C 11/10, A23L 1/22, A23G 1/32

(54) **Verbindungen zur Maskierung eines unangenehmen Geschmacks**

(71) Anmelder: Symrise AG, 37603 Holzminden (DE)
(72) Erfinder: Ley, Jakob, 37603 Holzminden (DE); Backes, Michael, 37603 Holzminden (DE); Wiedwald, Bernd, 37603 Holzminden (DE); Obst, Katja, 37603 Holzminden (DE)
(74) Vertreter: Fabry, Bernd

(57) **Zusammenfassung**

Die Erfindung betrifft neue Verbindungen und Salze von neuen Verbindungen, die zum Maskieren oder Vermindern eines unangenehmen Geschmackseindrucks eines unangenehm schmeckenden Stoffes geeignet sind, sowie deren Verwendung zum Maskieren oder Vermindern eines solchen unangenehmen Geschmackseindrucks. Hierbei sind die unangenehmen Geschmackseindrücke insbesondere solche, die als adstringierend, bitter, trocken, staubig, mehlig, kalkig und/oder metallisch bezeichnet werden. Ferner betrifft die Erfindung Zubereitungen, die eine wirksame Menge einer erfindungsgemäßen neuen Verbindung oder eines erfindungsgemäßen neuen Salzes umfassen, so dass der unangenehme Geschmackseindruck eines oder mehrerer gleichzeitig anwesender unangenehm schmeckender Stoffe maskiert oder vermindert wird.

## Beschreibung

Die Erfindung betrifft neue Verbindungen und Salze von neuen Verbindungen, die zum Maskieren oder Vermindern eines unangenehmen Geschmackseindrucks eines unangenehm schmeckenden Stoffes geeignet sind, sowie deren Verwendung zum Maskieren oder Vermindern eines solchen unangenehmen Geschmackseindrucks. Hierbei sind die unangenehmen Geschmackseindrücke insbesondere solche, die als adstringierend, bitter, trocken, staubig, mehlig, kalkig und/oder metallisch bezeichnet werden. Ferner betrifft die Erfindung Zubereitungen, die eine wirksame Menge einer erfindungsgemäßen neuen Verbindung oder eines erfindungsgemäßen neuen Salzes umfassen, so dass der unangenehme Geschmackseindruck eines oder mehrerer gleichzeitig anwesender unangenehm schmeckender Stoffe maskiert oder vermindert wird.

Nahrungs- oder Genussmittel enthalten häufig verschiedene adstringierend wirkende Stoffe (Adstringentien), die zwar einerseits in Maßen erwünscht und charakteristisch sind (z.B. Catechine in Tee oder Chlorogensäure in Kaffee), andererseits den Wert aber auch stark mindern können (z.B. Flavonoidglycoside und Limonoide in Zitrussäften, bitterer und/oder adstringierender Nachgeschmack vieler künstlicher Süßstoffe wie Aspartam, Rebaudiosid A, Steviosid oder Saccharin, hydrophobe Aminosäuren und/oder Peptide in Käse, Isoflavone und Proteine in Sojaprodukten).

Adstringierender Geschmack wird in der Regel durch Fällung von Prolin-reichen Proteinen im Speichel durch Adstringentien, z.B. Metallsalze oder Tannine, verursacht. Der normalerweise als "Schmiermittel" dienende homogene Speichel enthält durch diese Fällung vermehrt denaturierte Proteine, wodurch die Gleitfähigkeit des Speichels herabgesetzt wird und woraufhin ein als adstringierend empfundenes Gefühl im Mund verbleibt (Isabelle Lesschaeve und Ann C. Noble, American Journal of Clinical Nutrition 2005, 81, 330S-335S).

Insbesondere stark fettreduzierte Produkte (z.B. fettreduzierte oder ―freie Milchprodukte, insbesondere Joghurt oder Frischkäse sowie emulsionsbasierte Systeme wie Mayonnaise) zeigen oft ein reduziertes Mundgefühl.

Gemäß Verordnung EG (Nr. 1924/2006) sind seit dem 1. Juli 2007 als "fettarm" bezeichnete fettreduzierte Produkte feste Lebensmittel, die nicht mehr als 3 g Fett pro 100 g enthalten, bzw. flüssige Lebensmittel, die nicht mehr als 1,5 g Fett pro 100 ml enthalten. Ais "fettfrei" bezeichnete Lebensmittel dürfen nicht mehr als 0,5 g Fett pro 100 g bzw. 100 ml enthalten.

Insbesondere zur Senkung des natürlichen Gehalts an Adstringentien ist oft eine nachträgliche Behandlung nötig, beispielsweise durch einen zu verwerfenden Voraufguss bei grünem Tee zur Entfernung von Catechinen, oder enzymatisch, z.B. Behandlung von Tee mit zersetzenden Enzymen zur Zerstörung der adstringierenden Polyphenole, wie in WO 2003/022065 oder JP 2007-135481 beschrieben, oder Einsatz von speziellen Peptidasen bei der Reifung von Käse.

Diese Behandlungen zur Senkung des natürlichen Gehalts an Adstringentien sind jedoch belastend für das Produkt, erzeugen Abfallstoffe und bedingen z.B. auch Lösungsmittelreste und andere Rückstände (Enzyme) in den Produkten.

Daher besteht ein ständiger Bedarf an neuen Verbindungen, die in der Lage sind, unangenehme Geschmackseindrücke, insbesondere adstringierende, bittere, trockene, mehlige, staubige, kalkige und/oder metallische Geschmackseindrücke bereits in geringen Konzentrationen wirkungsvoll zu maskieren (vollständig zu unterdrücken) oder zumindest zu vermindern.

Insbesondere sind Stoffe gesucht, die in der Lage sind, in fettreduzierten Produkten, insbesondere Milchprodukten, Sojazubereitungen und Tees eingesetzt zu werden, sowie in protein- und tanninhaltigen Zubereitungen.

Besonders wichtig ist die Unterdrückung (Maskierung) unangenehmer Geschmackseindrücke auch bei vielen pharmazeutischen Wirkstoffen, da hierdurch die Bereitschaft der Patienten (insbesondere von Kindern) deutlich erhöht werden kann, eine den Wirkstoff enthaltende Zubereitung oral aufzunehmen. Viele pharmazeutische Wirkstoffe, beispielsweise Aspirin, Salicin, Paracetamol, Ambroxol oder Chinin, um nur eine sehr kleine Auswahl zur Verdeutlichung zu nennen, besitzen einen ausgeprägt adstringierenden und/oder metallischen Geschmack und/oder Nachgeschmack.

Herkömmlicherweise wurden als übliche Gegenmittel für Adstringentien Fettemulsionen eingesetzt, deren Verwendung jedoch in vielen Fällen nicht angezeigt ist, so z.B. in fettfreien Getränken oder gesundheitsbewussten, fettarmen Produkten.

In JP 2000-287630 und WO 2006/013930 wird eine Methode beschrieben, den adstringierenden Geschmack von Polyphenolen in Teegetränken unter Verwendung von Zuckern und bestimmten Aminosäuren zu mindern. Dabei werden die Aminosäuren in einer Menge von 0,04 bis 0,1 % eingesetzt.

In JP 2001-046037 wird die Minderung des adstringierenden Geschmacks von Polyphenolen durch den Einsatz von Stärke und Proteinen erreicht. Dieses Verfahren hat jedoch den Nachteil, dass insbesondere Proteine und Stärke großen Einfluss auf die rheologischen Eigenschaften der Produkte haben.

In JP 2003-128664 wird die Reduktion der Adstringenz durch Salzbildung der Polyphenole erreicht. Leider wird dadurch in vielen Fällen die Stabilität dieser Verbindungen gemindert und die Oxidationsanfälligkeit derselben gesteigert.

In JP 2004-315441 wird beschrieben, dass man den adstringierenden Geschmackseindruck von Eisensalzen mit speziellen Aminosäuren wie gamma-Aminobuttersäure reduzieren kann. Durch den notwendigen Einsatz extrem hoher Konzentrationen (1 % und höher) erhält das Produkt bzw. die Zubereitung jedoch einen sauren Geschmack. Spezielle Zucker wie Palatinose, auch in Kombination mit Süßstoffen, wurden zur Maskierung der Adstringenz von Sojagetränken vorgeschlagen (WO 2004/062385), allerdings sind auch hier sehr große Mengen erforderlich. In WO 2005/016031 werden nichtreduzierende Disaccharide wie Trehalose als Adstringenz-Maskierer vorgeschlagen. In JP 2004-337132 wird die Maskierung mit Cyclofructanen beschrieben, die die adstringierenden Stoffe durch Komplexierung maskieren. Gemäß JP 2005-145933 sind größere Pektin- oder Alginatkonzentrationen (> 0,1 %) ebenfalls in der Lage, die Adstringenz von Polyphenolen z.B. aus Traubenkernextrakten zu reduzieren. Gallat-Catechine, wie sie z.B. in Grüntee vorkommen, wurden ebenfalls mit dieser Methode maskiert (N. Hayashi, T. Ujihara und K. Kohata, Biosci. Biotechnol. Biochem. 2005, 69 (7), 1306-1310).

Teilweise werden auch Süßstoffe zur Reduktion der Adstringenz eingesetzt, wie z.B, in JP 2007/135481 beschrieben. Dabei erhält man aber grundsätzlich nur süße Applikationen.

Bei den oben erwähnten und bekannten Verfahren besteht regelmäßig ein Nachteil darin, dass die Maskierungsmittel in erheblichen Mengen (> 0,05 %) eingesetzt werden sollten, was sowohl zu Kosten- als auch zu Anwendungsproblemen im Falle polymerer Kohlenhydrate oder Proteine führen kann.

In US 2009/124701 (EP 08 168 519) wird die Verwendung unpolarer, langkettiger und ungesättigter Alkamide wie z.B. 2*E*,4*E*-Decadiensäure-*N*-isobutylamid (*trans*-Pellitorin) in geringen Mengen als wirksame Maskierer für Adstringentien beschrieben. Dabei sind die beschriebenen Alkamide gemäß US 2009/124701 (EP 08 168 519) aber regelmäßig Verbindungen, die einen starken chemesthetischen Reiz (wie z.B. tingling, wärmend, scharf, kühlend, stechend oder beißend) verursachen, so dass ein Einsatz in höheren Konzentrationen nicht mehr wirksam die Adstringenz verhindern kann, ohne die oben genannten weiteren Geschmackseffekte zu zeigen.

WO 2009/141294 offenbart Acylaminocarbonsäuren, welche eine cis-konfigurierte Doppelbindung besitzen und einen Geschmackseindruck hervorrufen, der als "tingling" beschrieben wird. Der vermittelte Geschmackseindruck umfasst dabei keinen brennenden Aspekt.

Es war daher die primäre Aufgabe der vorliegenden Erfindung, Substanzen (Stoffe oder Stoffgemische) anzugeben, die
(a) bereits in geringen Mengen zur Maskierung (vollständigen Unterdrückung) oder zumindest zur Verminderung des unangenehmen Geschmackseindrucks unangenehm schmeckender Stoffe geeignet sind, wobei zu den unangenehmen Geschmackseindrücken die Geschmackseindrücke adstringierend, bitter, trocken, staubig, mehlig, kalkig sowie metallisch zählen, wobei die anzugebende Substanz insbesondere einen Adstringenz-maskierenden Effekt gegen eine Vielzahl von Adstringentien vorzugsweise zeigen sollte,
(b) breit anwendbar sind, d.h. in einer Vielzahl von Zubereitungen eingesetzt werden können, ohne in diesen einen nachteiligen Effekt zu bewirken,
(c) synthetisch leicht zugänglich sind und
(d) in üblichen Einsatzkonzentrationen keinen chemesthetischen oder sonstigen negativen Geschmackseindruck verursachen.

Weitere Aufgaben der vorliegenden Erfindung ergeben sich aus der nachfolgenden Beschreibung, den Ausführungsbeispielen und den beigefügten Patentansprüchen.

Die primär gestellte Aufgabe wird gelöst durch die Verbindungen der Formel (1) 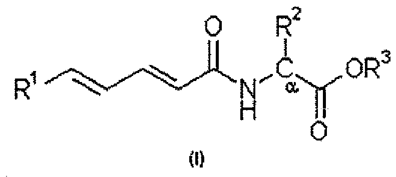 wobei
R¹ eine Alkylgruppe mit 1 bis 6 Kohlenstoffatomen bedeutet,
und
R² Wasserstoff oder eine organische Gruppe mit 1 bis 10 Kohlenstoffatomen bedeutet und
R³ Wasserstoff oder eine Alkylgruppe mit 1 bis 4 Kohlenstoffatomen bedeutet,
sowie
die pharmazeutisch akzeptablen Salze von Verbindungen der Formel (I), in denen R² eine Hydroxy- oder Carboxylgruppe umfasst und/oder R³ Wasserstoff ist.

Die Verbindungen der Formel (1) besitzen im Vergleich zu den aus US 2009/124701 bekannten Verbindungen eine die Polarität erhöhende Carboxylfunktion. Wenngleich dies durch theoretische Überlegungen nicht abschließend erklärt werden kann, scheint es doch so zu sein, dass diese zusätzliche polare Gruppe verantwortlich oder zumindest mitverantwortlich dafür ist, dass eine erfindungsgemäße Verbindung der Formel (1) hervorragend zum Maskieren oder zumindest Vermindern eines unangenehmen Geschmackseindrucks eines unangenehm schmeckenden Stoffes geeignet ist, ohne dabei (wie die aus US 2009/124701 bekannten Verbindungen) selbst einen nachteiligen chemesthetischen Reiz zu vermitteln. Dies gilt analog für die erfindungsgemäßen Salze.

Die vorliegende Erfindung betrifft auch die Verwendung einer einzelnen erfindungsgemäßen Verbindung oder eines einzelnen erfindungsgemäßen Salzes und/oder einer Mischung umfassend oder bestehend aus zwei oder mehr unterschiedlichen erfindungsgemäßen Verbindungen oder erfindungsgemäßen Salzen als nicht-chemesthetischer Geschmacksmaskierer. Der Begriff "nicht-chemesthetischer Geschmacksmaskierer" bezeichnet dabei Substanzen, die in ihrer Einsatzkonzentration keinen chemesthetischen Reiz verursachen, aber den unangenehmen Geschmack eines oder mehrerer unangenehm schmeckender Stoffe maskieren oder zumindest reduzieren.

Besonders bevorzugt bedeutet R² in erfindungsgemäßen Verbindungen oder Salzen Wasserstoff oder eine organische Gruppe mit 1 bis 10 Kohlenstoffatomen, die ausgewählt ist aus der Gruppe bestehend aus verzweigtes Alkyl, unverzweigtes Alkyl, Carboxyalkyl, Alkoxycarbonyl, Carbamoylalkyl, Aminoalkyl, Sulfanylalkyl, Alkylsulfanylalkyl, Hydroxyalkyl, Guanidinylalkyl, Heterozyklylalkyl, unsubstituiertes Arylalkyl und Hydroxyarylalkyl.

Bevorzugte organische Gruppen für R² in der erfindungsgemäßen Verbindung der Formel (1) sind beispielsweise:
verzweigte und unverzweigte Alkyle, z.B.: ―CH₃, ―CH₂―CH₃, ―CH(CH₃)₂, ―CH₂― CH(CH₃)₂, ―CH(CH₃)-CH₂―CH₃,
Carboxyalkyle, z.B.: ―CH₂―CH₂-COOH, ―CH₂―COOH,
Alkoxycarbonyle, z.B.: ―CH₂―CH₂-COOCH₃, ―CH₂―COOCH₃
Carbamoylalkyle, z.B.: ―CH₂―CH₂-CONH₂, ―CH₂―CONH₂,
Aminoalkyle, z.B.: ―CH₂―CH₂―CH₂―NH₂, ―(CH₂)₄―NH₂,
Sulfanylalkyle, z.B.: ―CH₂―SH, ―CH₂―CH₂―SH,
Alkylthioalkyle, z.B.: ―CH₂―CH₂―S―CH₃,
Hydroxyalkyle, z.B.: ―CH₂―OH, ―CH₂―CH₂―OH, ―CH(OH)―CH₃,
Guanidinylalkyle, z.B.: ―CH₂―CH₂―CH₂―NH―(C=NH)―NH₂,
Heterozyklylalkyle, z.B.: ―CH₂-3-Indolyl, ―CH₂-4-Imidazolyl,
Arylalkyle, z.B.: ―CH₂-Ph,
Hydroxyarylalkyle, z.B.: ―CH₂-(Ph-4-OH).

Besonders bevorzugt sind erfindungsgemäße Verbindungen bzw. Salze, in denen die Reste R¹, R² und/oder R³ auf besondere Weise ausgewählt sind. Vorzugsweise ist R¹ ausgewählt aus der Gruppe bestehend aus Methyl, Ethyl, 1-Propyl, 2-Propyl, 1-Butyl, 2-Butyl, 1-Pentyl, 2-Pentyl, 3-Pentyl, 1-Hexyl, 2-Hexyl, 3-Hexyl, 2-Methylprop-1-yl, 2-Methylprop-2-yl, 2-Methylbut-1-yl, 3-Methylbut-1-yl, 2-Methylbut-2-yl, 3-Methylbut-2-yl, 2-Methylpent-1-yl, 2-Methylpent-2-yl, 2-Methylpent-3-yl, 3-Methylpent-1-yl, 3-Methylpent-2-yl, 3-Methylpent-3-yl, 4-Methylpent-1-yl und 4-Methylpent-2-yl.

Alternativ oder (vorzugsweise) gleichzeitig ist R² vorzugsweise ausgewählt aus der Gruppe bestehend aus H, ―CH₃, ―CH₂―CH₃, ―CH(CH₃)₂, ―CH₂―CH(CH₃)₂, ―CH(CH₃)-CH₂―CH₃, ―CH₂―CH₂-COOCH₃, ―CH₂―CH₂-CONH₂, ―CH₂―CH₂-COOH, ―CH₂―COOCH₃, ―CH₂―CONH₂, ―CH₂―COOH, ―CH₂―OH, ―CH₂―CH₂―OH, ―CH(OH)―CH₃, -CH₂-Ph, -CH₂-(Ph-4-OH), -CH₂₋3-Indolyl, -CH₂-4-Imidazolyl, -CH₂-SH, -CH₂-CH₂-S-CH₃, -CH₂-CH₂-SH, - CH₂-CH₂-CH₂-NH₂, -(CH₂)₄-NH₂ und -CH₂-CH₂-CH₂-NH-(C=NH)-NH₂.

Alternativ oder (vorzugsweise) gleichzeitig ist R³ vorzugsweise ausgewählt aus der Gruppe bestehend aus Wasserstoff, Methyl, Ethyl, 1-Propyl, 2-Propyl, 1-Butyl, 2-Butyl, 2-Methylprop-1-yl und 2-Methylprop-2-yl.

Aus dem Vorgesagten ergibt sich, dass es besonders bevorzugt ist, sämtliche der Gruppen R¹, R² und R³ jeweils aus den vorgenannten Listen auszuwählen.

Für jede der Gruppen R¹, R² und R³ gibt es wiederum bevorzugte Bedeutungen, die besonders bevorzugte erfindungsgemäße Verbindungen bzw. Salze definieren. Besonders bevorzugt ist R¹ ausgewählt aus der Gruppe bestehend aus Methyl, Ethyl, 1-Propyl, 1-Butyl, 1-Pentyl, 1-Hexyl, 2-Methylprop-1-yl-, 2-Methylbut-1-yl, 3-Methylbut-1-yl, 2-Methylpent-1-yl, 3-Methylpent-1-yl und 4-Methylpent-1-yl.

Alternativ oder (vorzugsweise) gleichzeitig ist R² besonders bevorzugt ausgewählt aus der Gruppe bestehend aus H, ―CH₃,―CH₂―CH₃, ―CH(CH₃)₂, ―CH₂―CH(CH₃)₂, ―CH(CH₃)-CH₂―CH₃, ―CH₂―CH₂-COOCH₃, ―CH₂―CH₂―COOH, ―CH₂―COOCH₃, ―CH₂―COOH, - CH₂―OH, ―CH₂―CH₂―OH, ―CH(OH)―CH₃.

Alternativ oder (vorzugsweise) gleichzeitig ist R³ bevorzugt ausgewählt aus der Gruppe bestehend aus Wasserstoff, Methyl und Ethyl.

Besonders bevorzugt ist R¹ ausgewählt aus der Gruppe bestehend aus Methyl, Ethyl, 1-Propyl, 1-Butyl, 1-Pentyl und 1-Hexyl.

Alternativ oder (vorzugsweise) gleichzeitig ist dabei R² besonders bevorzugt ausgewählt aus der Gruppe bestehend aus H, ―CH₃, ―CH₂―CH₃, ―CH(CH₃)₂, ―CH₂-CH(CH₃)₂, ― CH(CH₃)-CH₂-CH₃, ―CH₂―CH₂-COOCH₃, ―CH₂―CH₂-COOH, ―CH₂―COOCH₃ und ―CH₂― COOH.

Alternativ oder (vorzugsweise) gleichzeitig ist dabei R³ bevorzugt ausgewählt aus der Gruppe bestehend aus Wasserstoff, Methyl und Ethyl.

Besonders bevorzugte erfindungsgemäße Verbindungen besitzen eine (2*E*,4*E*)-Deca-2,4-dienoylamino-Gruppe.

Ganz besonders bevorzugt sind die folgenden erfindungsgemäßen Verbindungen:
[(2*E*,4*E*)-Deca-2,4-dienoylamino]essigsäuremethylester (Verbindung 1)
[(2*E*,4*E*)-Deca-2,4-dienoylamino]essigsäure (Verbindung 2)
(2*S*)-2-[(2*E*,4*E*)-Deca-2,4-dienoylamino]propionsäuremethylester (Verbindung 3)
(2*S*)-2-[(2*E*,4*E*)-Deca-2,4-dienoylamino]propionsäure (Verbindung 4)
(2*S*)-2-[(2*E*,4*E*)-Deca-2,4-dienoylamino]pentandicarbonsäuredimethylester (Verbindung 5)
(2*S*)-2-[(2*E*,4*E*)-Deca-2,4-dienoylamino]pentandicarbonsäure (Verbindung 6)

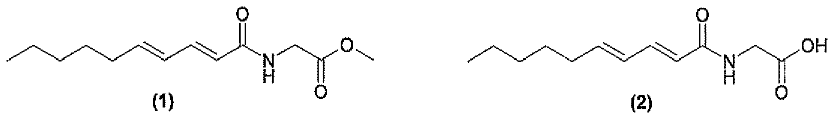

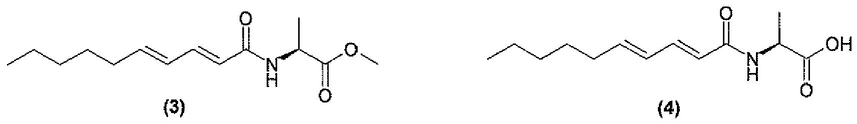

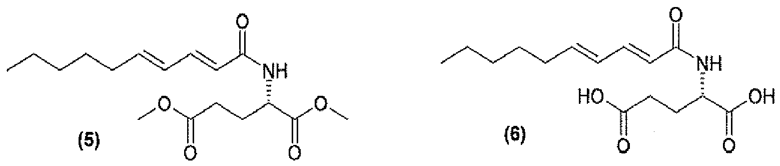

Besonders bevorzugt sind erfindungsgemäße Verbindungen oder Salze, wobei in der Formel (I) R² eine organische Gruppe mit 1 bis 10 Kohlenstoffatomen bedeutet, das alpha-C-Atom chiral ist und die Konfiguration am alpha-C-Atom derjenigen der korrespondierenden L-alpha-Aminosäure H₂N-CR²H-COOH entspricht. Solche Verbindungen bzw. deren Salze lassen sich aus den korrespondierenden L-alpha-Aminosäuren ohne Änderung der Konfiguration am alpha C-Atom synthetisieren. Beispielsweise läßt sich (2*S*)-2-[(2*E*,4*E*)-Deca-2,4-dienoylamino)propionsäuremethylester (Verbindung 3) ausgehend von L-Alaninmethylester synthetisieren (siehe Synthesebeispiel). Auch erfindungsgemäße Verbindungen oder Salze, wobei in der Formel (I) R² Wasserstoff bedeutet, lassen sich ausgehend von der korrespondierenden Aminosäure synthetisieren, z.B. [(2*E*,4*E*)-Deca-2,4-dienoylamino]essigsäuremethylester (Verbindung 1) ausgehend von Glycin.

Ganz besonders bevorzugt sind erfindungsgemäße Verbindungen oder Salze, die ausgehend von einer proteinogenen Aminosäure ohne Änderung der Konfiguration am alpha C-Atom synthetisierbar sind (bei chiralem alpha-C-Atom handelt es sich bei den geeigneten Startmaterialien um proteinogene L-alpha-Aminosäuren).

Erfindungsgemäße pharmazeutisch akzeptable Salze einer erfindungsgemäßen Verbindung der Formel (I) basieren auf einer Verbindung der Formel (I), wobei R² eine Hydroxy- oder Carboxylgruppe umfasst und/oder R³ Wasserstoff ist. Vorzugsweise ist ein erfindungsgemäßes pharmazeutisch akzeptables Salz ausgewählt aus der Gruppe bestehend aus Natrium-, Kalium-, Ammonium-, Magnesium-, Calcium- und Zinksalz.

Die erfindungsgemäßen Verbindungen sowie die erfindungsgemäßen Salze sind im Stand der Technik noch nicht beschrieben worden. Offenbart wurden lediglich bestimmte 2,4-Alkandiensäureamide im strukturell weiter entfernten Umfeld:
2-[(2*E*,4*E*)-Dodeca-2,4-dienoylamino]butansäure wurde in US 4742047 (Bristol-Myers) als Zwischenprodukt für eine Synthese offenbart. [(2*E*,4*E*)-Tetradeca-2,4-dienoylamino]essigsäure wurde in JP 08-073361 bzw. in EP 525479 ebenfalls als Synthesezwischenprodukt beschrieben. DE 36 29 465 offenbart unter anderem die Verbindung (2*E*,4*E*)-N-(1-oxo-2,4-dodecadienyl)-L-threonin.

Keines der genannten Dokumente offenbart einen geschmacksmaskierenden Effekt oder eine Anwendung der offenbarten Zwischenprodukte als Aroma- oder Geschmackstoff oder Nahrungs- oder Genussmittel.

In eigenen sensorischen Untersuchungen hat sich herausgestellt, dass die erfindungsgemäßen Verbindungen und Salze in einzigartiger Weise dazu geeignet sind, einen adstringierenden Geschmack bzw. adstringierenden Nachgeschmack eines unangenehm schmeckenden Stoffes zu maskieren oder zumindest zu vermindern, ohne dabei selber einen signifikanten chemesthetischen Reiz zu verursachen.

Die vorliegende Erfindung betrifft entsprechend auch die Verwendung einer einzelnen erfindungsgemäßen Verbindung oder eines einzelnen erfindungsgemäßen Salzes und/oder einer Mischung umfassend oder bestehend aus zwei oder mehr unterschiedlichen erfindungsgemäßen Verbindungen oder erfindungsgemäßen Salzen zum Maskieren oder Vermindern eines unangenehmen Geschmackseindrucks eines unangenehm schmeckenden Stoffes.

Der unangenehme Geschmackseindruck ist dabei vorzugsweise ausgewählt aus der Gruppe bestehend aus adstringierend, bitter, trocken, staubig, mehlig, kalkig und metallisch, wobei es im Rahmen der vorliegenden Erfindung besonders bevorzugt ist, wenn die besagte erfindungsgemäße Verbindung, das besagte erfindungsgemäße Salz oder die besagte Mischung eingesetzt wird, um einen adstringierenden Geschmackseindruck zu maskieren oder zumindest zu vermindern.

Der Begriff "Geschmackseindruck" umfasst im Rahmen des vorliegenden Textes auch immer die durch einen Nachgeschmack bedingten Geschmackseindrücke. Entsprechend umfasst der Begriff "Geschmack" auch den Nachgeschmack.

Unangenehm schmeckende Stoffe im Sinne des vorliegenden Textes sind Stoffe, die einen adstringierenden, bitteren, trockenen, staubigen, mehligen, kalkigen und/oder metallischen Geschmack oder Nachgeschmack besitzen. Ein adstringierender Geschmackseindruck geht dabei oftmals einher mit einem oder mehreren der Geschmackseindrücke bitter, trocken, staubig, mehlig, kalkig und/oder metallisch.

Die Geeignetheit der erfindungsgemäßen Verbindung oder eines einzelnen erfindungsgemäßen Salzes und/oder einer Mischung umfassend oder bestehend aus zwei oder mehr unterschiedlichen erfindungsgemäßen Verbindungen oder erfindungsgemäßen Salzen zum Maskieren oder Vermindern eines unangenehmen Geschmackseindrucks eines unangenehm schmeckenden Stoffes lässt sich in üblicher Weise mittels Paneltests ermitteln. Besonders überraschend war die in eigenen Untersuchungen gewonnene Erkenntnis, dass die Verbindungen der Formel (I) (bzw. ihre Salze) keinen oder nur einen extrem schwachen Tingling-Effekt verursachen, dabei aber dennoch in der Lage sind, die Adstringenz von z.B. Teecatechinen deutlich zu reduzieren. Diese Erkenntnis war insbesondere deshalb überraschend, weil die Fachleute aus den in US 2009/124701 offenbarten Ergebnissen zunächst schlossen, dass eine wesentliche Ursache für die Maskierung der Adstringenz der dort beschriebene Tingling-Effekt ist.

Die vorgenannten, unangenehm schmeckenden Stoffe können noch weitere, in der Regel nicht unangenehme Geschmacks- und/oder Geruchsqualitäten besitzen. Als weitere, im Sinne der vorliegenden Erfindung nicht unangenehme Geschmacksqualitäten sind z.B. die Eindrücke würzig, umami, süß, salzig, sauer zu nennen.

Ausgewählte Beispiele unangenehm schmeckender Stoffe im Sinne der Erfindung sind weiter unten genannt.

Wie bereits erwähnt, betrifft die vorliegende Erfindung gemäß einem ihrer Aspekte die Verwendung einer einzelnen erfindungsgemäßen Verbindung, eines einzelnen erfindungsgemäßen Salzes oder einer entsprechenden Mischung zum Maskieren oder Vermindern eines unangenehmen Geschmackseindrucks eines unangenehm schmeckenden Stoffes. Dem entspricht die erfindungsgemäße Verwendung als nicht chemesthetischer Geschmacksmaskierer. Vorzugsweise wird die erfindungsgemäß zu verwendende Verbindung bzw. das erfindungsgemäß zu verwendende Salz bzw. die entsprechende Mischung in einer Zubereitung eingesetzt. Entsprechend ist eine erfindungsgemäße Verwendung bevorzugt, in der die genannten Substanzen (Verbindung, Salz bzw. Mischung) in einer zur oralen Aufnahme bestimmten pharmazeutischen oder einer der Ernährung, der Mundpflege oder dem Genuss dienenden Zubereitung vorliegen.

Bevorzugte Zubereitungen, in denen die erfindungsgemäßen Verbindungen bzw. Salze oder entsprechende Mischungen eingesetzt werden, sind Zubereitungen, die auf Soja oder Tee (bevorzugt *Camellia-* insbesondere Grün-, Schwarz-, Gelb-, weiße Oolong-Teeextrakte) basieren oder bei denen es sich um fettreduzierte (low-fat) Milchprodukte wie zum Beispiel fettreduzierte Joghurts handelt. Zusätzlich besonders bevorzugte Zubereitungen sind Zubereitungen, die isolierte, native oder partiell hydrolisierte und/oder angereicherte pflanzliche Proteine, insbesondere Leguminosen-Proteine, z.B. Azukibohnenprotein, Bohnenprotein, Erbsenprotein, Kleeprotein, Alfalfa-Protein, Lupinen-Protein enthalten, sowie Zubereitungen, die Tannine, insbesondere Vitis-, Blätter-, Stängel-, Trauben-, Traubenschalen-, Wein- oder Traubenkernextrakte enthalten.

Auf Soja basierende Zubereitungen bzw. Produkte im Sinne der Erfindung sind insbesondere Produkte aus Sojaprotein oder anderen Sojabohnen-Fraktionen, wie z. B. Sojamilch und daraus gefertigte Produkte, Getränke enthaltend isoliertes oder enzymatisch behandeltes Sojaprotein, Sojamehl, Sojalezithin-haltige Zubereitungen, fermentierte Produkte wie Tofu oder Tempé oder daraus gefertigte Produkte und Mischungen mit Fruchtzubereitungen und fakultativ Aromen.

Auf Tee basierende Zubereitungen, in denen die erfindungsgemäßen Verbindungen bzw. Salze bzw. Mischungen vorteilhaft eingesetzt werden können, umfassen vorzugsweise einen Anteil aus Teilen oder Extrakten, bevorzugt Wasserextrakten aus Pflanzenteilen von *Camellia ssp.* verschiedener Fermentationsstufen. Solche Zubereitungen auf Tee-Basis werden z.B. als weißer, gelber, grüner, roter (Puh-Err-), Oolong- oder schwarzer Tee hergestellt und enthalten die als besonders adstringierend geltenden Polyphenole, insbesondere Catechine und deren Derivate und Abbauprodukte. Entsprechende Zubereitungen können in Form von Teeblattzubereitungen, Teeextrakt oder Teekonzentraten in konsumierbaren Lebensmitteln oder Halbfertigprodukten eingesetzt werden. Selbstverständlich können auch andere Teeprodukte im Sinne der Erfindung verwendet werden, so z.B. Mate-Tee (*Ilex ssp.*), Rotbusch (*Aspalanthus ssp.*) oder Honigbuschtee (*Cyclopia ssp.*) oder weitere Kräutertees, die Catechine oder Tannine enthalten. Vorzugsweise wird eine einzelne erfindungsgemäße Verbindung oder ein einzelnes erfindungsgemäßes Salz bzw. eine entsprechende Mischung zum Maskieren oder Vermindern eines unangenehmen Geschmackseindrucks eines unangenehm schmeckenden Stoffes in einer Zubereitung eingesetzt, welche Polyphenole, insbesondere Catechine, oder deren Derivate bzw. Abbauprodukte enthält. Es ergibt sich aus dem Vorgesagten, dass Polyphenole (insbesondere Catechine) übliche Bestandeile bestimmter Tees sind. Low-fat-Milchprodukte sind fettarme Produkte im Sinne der weiter oben erwähnten EG-Verordnung.

Eine erfindungsgemäße Zubereitung umfasst
a)
   - eine erfindungsgemäße Verbindung oder ein erfindungsgemäßes Salz wie oben definiert (vorzugsweise wie vorstehend als bevorzugt bezeichnet)
      und/oder
   - eine Mischung umfassend oder bestehend aus zwei oder mehr unterschiedlichen erfindungsgemäßen Verbindungen oder Salzen wie oben definiert (vorzugsweise wie vorstehend-als-bevorzugt bezeichnet)
      und zusätzlich
b)
   - ein oder mehrere unangenehm schmeckende Stoffe (vorzugsweise wie oben oder unten beschrieben),
      wobei
   - die Menge des bzw. der unangenehm schmeckenden Stoffe ausreicht, um in einer Vergleichszubereitung, die keine erfindungsgemäße Verbindung und kein erfindungsgemäßes Salz umfasst, aber ansonsten identisch zusammengesetzt ist, als unangenehmer Geschmack (wie oben beschrieben) wahrgenommen zu werden, und wobei
   - die Menge der erfindungsgemäßen Verbindung, des erfindungsgemäßen Salzes oder der erfindungsgemäßen Mischung in der Zubereitung ausreicht, um im Vergleich mit der Vergleichszubereitung den unangenehmen Geschmackseindruck des unangenehm schmeckenden Stoffes oder Stoffgemisches sensorisch zu maskieren oder zu vermindern.

Es versteht sich, dass sämtliche Ausführungen, die weiter oben zu bevorzugten erfindungsgemäßen Verbindungen bzw. Salzen oder zu erfindungsgemäßen Verwendungen gemacht wurden, für die erfindungsgemäßen Zubereitungen entsprechend zutreffen.

Es wurde bereits weiter oben ausgeführt, dass die erfindungsgemäßen Verbindungen bzw. Salze selbst keinen bzw. einen nur äußerst schwachen chemesthetischen Geschmackseindruck vermitteln. Es versteht sich, dass deshalb auch erfindungsgemäße Zubereitungen bevorzugt sind, die ebenfalls keinen solchen chemesthetischen Geschmackseindruck vermitteln. Selbstverständlich ist es aber auch durchaus möglich, ganz bewusst durch Zugabe von ausgewählten Stoffen bzw. Substanzen mit chemesthetischen Eigenschaften einen chemesthetischen Geschmackseindruck gezielt hervorzurufen, wobei die erfindungsgemäßen Verbindungen bzw. Salze selbst keinen bzw. einen nur vernachlässigbaren Beitrag leisten.

Eine erfindungsgemäße Zubereitung ist vorzugsweise eine solche, in der der sensorisch maskierte oder verminderte unangenehme Geschmackseindruck des unangenehm schmeckenden Stoffes oder Stoffgemisches einen, zwei, drei, vier, fünf, sechs oder sämtliche der Geschmackseindrücke umfasst, die ausgewählt sind aus der Gruppe bestehend aus adstringierend, bitter, trocken, staubig, mehlig, kalkig und metallisch.

Mit anderen Worten sind erfindungsgemäße Zubereitungen bevorzugt, in denen die erfindungsgemäße Verbindung bzw. das erfindungsgemäße Salz bzw. die entsprechende Mischung ihre im Zentrum der vorliegenden Erfindung stehende Aufgabe löst, d.h. den unangenehmen Geschmackseindruck einer unangenehm schmeckenden Substanz maskiert oder zumindest vermindert.

Unangenehm schmeckende Stoffe, die in einer erfindungsgemäßen Zubereitung neben einer erfindungsgemäßen Verbindung, einem erfindungsgemäßen Salz bzw. einer erfindungsgemäßen Mischung vorliegen, sind vorzugsweise ausgewählt aus der Gruppe bestehend aus: Xanthinalkaloide, Xanthine, Alkaloide, phenolische Glycoside, Flavonoidglycoside, Chalcone oder Chalconglycoside, hydrolysierbare Tannine, nicht-hydrolysierbare Tannine, Flavone, Phenole, Polyphenole, terpenoide Bitter- und Gerbstoffe, Absinthin aus Wermut, Amarogentin aus Enzian, metallische Salze, pharmazeutische Wirkstoffe, Vitamine, Denatoniumbenzoat, Sucraloseoctaacetat, Eisensalze, Aluminiumsalze, Zinksalze, Harnstoff, ungesättigte Fettsäuren, Aminosäuren, Peptide, Proteine, Saponine und Isoflavonoide.

Es hat sich in eigenen Untersuchungen überraschenderweise gezeigt, dass die erfindungsgemäßen Verbindungen bzw. Salze bzw. deren Mischungen in Kombination mit Aromastoffen, die einen milchig-sahnigen und Mundfülle gebenden und/oder einen süß-karamelligen Geschmackseindruck vermitteln, besonders gut den unangenehmen Geschmack eines wie oben definierten unangenehm schmeckenden Stoffes maskieren oder zumindest vermindern. Eine bevorzugte erfindungsgemäße Zubereitung umfasst somit neben den weiter oben genannten Bestandteilen (vorzugsweise Bestandteile, die weiter oben als bevorzugt bezeichnet sind), zusätzlich einen oder mehrere Aromastoffe, die einen milchig-sahnigen und Mundfülle gebenden und/oder einen süß-karamelligen Geschmackseindruck vermitteln. Vorzugsweise umfasst eine derartige, bevorzugte Zubereitung zudem auch ein oder mehrere Lösungsmittel, feste oder flüssige Trägerstoffe, Hilfsstoffe und/oder Stabilisierungsmittel.

Bevorzugt können die erfindungsgemäßen Zubereitungen auch eine Aromakomposition (d.h. eine Komposition mehrerer Aromastoffe) enthalten, um den Geschmack und/oder Geruch der Zubereitung abzurunden und zu verfeinern. Geeignete Aromakompositionen enthalten z.B. synthetische, natürliche oder naturidentische Aroma-, Riech- und Geschmacksstoffe sowie geeignete Hilfs- und Trägerstoffe.

Als weitere Bestandteile für erfindungsgemäße Zubereitungen können übliche Grund-, Hilfs- und Zusatzstoffe für Nahrungs- oder Genussmittel verwendet werden. Einige dieser Substanzen besitzen einen unangenehmen Geschmack im Sinne der eingangs gegebenen Definition.

Weitere übliche Wirk-, Grund-, Hilfs- und Zusatzstoffe für die der Ernährung, der Mundpflege oder dem Genuss dienenden Zubereitungen können in Mengen von 5 bis 99,999999 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,999999 Gew.-%, vorzugsweise 5 bis 80 Gew.-%, bezogen auf das Gesamtgewicht der Zubereitung, aufweisen.

Beispiele üblicher Grund-, Hilfs- und Zusatzstoffe für erfindungsgemäße Zubereitungen sind Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine wie Maltodextrin und Dextrose, Amylose, Amylopektin, Inulin, Xylane, Cellulose), Zuckeralkohole (z.B. Sorbit), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. Taurin), Peptide, native oder prozessierte Proteine (z.B. Gelatine), Enzyme (z.B. Peptidasen), Nukleinsäuren, Nucleotide, andere als die erfindungsgemäß verwendeten Geschmackskorrigenzien für unangenehme Geschmackseindrücke (z. B. Hesperetin, Phloretin oder andere gemäß EP 1 972 203 A1 zu verwendende Hydroxychalkonderivate sowie gegebenenfalls die dort beschriebenen Lactone), Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole), Stabilisatoren (z.B. Carageenan, Alginat), Trägerstoffe (z.B. Triacetin, 1,2-Propylenglycol, Maltodextrin und Dextrose), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), zusätzliche Bitterstoffe (z.B. Chinin, Coffein, Limonin, Amarogentin, Humolone, Lupolone, Catechine, Tannine), Süßstoffe (z.B. Saccharin, Cyclamat, Aspartam, Neotam, Stevioside, Rebaudioside, Acesulfam K, Neohesperidindihydrochalkon, Thaumatin, Superaspartam), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), ätherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal (das heißt scharfe, stechende, prickelnde, kratzende, adstringierende, Wärme- oder Kälte-Effekte verursachende) wirksame Stoffe oder Pflanzenextrakte, enthaltend solche trigeminal wirksamen Stoffe, synthetische, natürliche oder naturidentische Aromastoffe oder Riechstoffe sowie Geruchskorrigenzien.

Selbstverständlich gilt hinsichtlich der bevorzugt einzusetzenden erfindungsgemäßen Verbindungen sowie hinsichtlich der unangenehm schmeckenden Stoffe das jeweils weiter oben Gesagte entsprechend. Hinsichtlich bevorzugter Aromastoffe, die einen milchig-sahnigen und Mundfülle gebenden und/oder einen süß-karamelligen Geschmackseindruck vermitteln, sind weiter unten Beispiele angegeben.

Eine bevorzugte erfindungsgemäße Zubereitung ist eine zur oralen Aufnahme bestimmte pharmazeutische oder eine der Ernährung, der Mundpflege oder dem Genuss dienenden Zubereitung. Unter den der Ernährung dienenden Zubereitungen sind wiederum auf Soja basierende Produkte, Teezubereitungen und fettreduzierte Milchprodukte (insbesondere low-fat Joghurts) bevorzugt.

Hinsichtlich einzelner erfindungsgemäßer Verbindungen der Formel (I) besteht die Vermutung, dass diese in größerer Konzentration den Speichelfluss verstärken. In erfindungsgemäßen Zubereitungen ist die Gesamtmenge an erfindungsgemäßen Verbindungen bzw. Salzen vorzugsweise so bemessen, dass sie nicht ausreicht, beim Verzehr den Speichelfluss zu verstärken.

Besonders überraschend ist, dass in den erfindungsgemäßen Zubereitungen bevorzugt einzusetzende Aromastoffe die positive Eigenschaft aufweisen, die Wirkung von erfindungsgemäßen Verbindungen bzw. Salzen oder deren Mischungen, d.h. deren Wirkung (einen unangenehmen Geschmackseindruck zu maskieren oder zu vermindern) zu verstärken. In einer erfindungsgemäßen Zubereitung werden deshalb vorzugsweise bevorzugte Aromastoffe (wie nachfolgend angegeben) in einer solchen Menge eingesetzt, dass die den unangenehmen Geschmackseindruck maskierende bzw. vermindernde Eigenschaft der erfindungsgemäßen Verbindungen bzw. Salze (synergistisch) verstärkt wird.

Die auffallend starke Verminderung bzw. Maskierung des adstringierenden Geschmacks eines unangenehm schmeckenden Stoffes, der einen adstringierenden Geschmackseindruck vermittelt, insbesondere in auf Soja basierenden Produkten und fettreduzierten Joghurts, war in eigenen Untersuchungen besonders überraschend. Auch insoweit war der weiter oben beschriebene verstärkende Effekt zu beobachten, der aus der Kombination einer erfindungsgemäßen Verbindung bzw. eines erfindungsgemäßen Salzes mit dem oder den nachfolgend angegebenen Aromastoffen resultiert.

Als besonders wirksam in der Verstärkung der geschmacksmaskierenden bzw. geschmacksvermindernden Eigenschaften von erfindungsgemäßen Verbindungen bzw. Salzen wurden bereits Aromastoffe genannt, die einen milchig-sahnigen und Mundfülle gebenden Geschmackseindruck vermitteln (hierzu gehören z. B. Diacetyl, Acetoin oder delta-Lactone) sowie Aromastoffe, die einen süß-karamelligen Geschmackseindruck vermitteln, der vorzugsweise ein Zuckerprofil unterstützt (hierzu gehören z. B. Maltol, Vanillin, Benzaldehyd, Furaneol, Heliotropin).

Im Einzelnen sind zur Kombination mit erfindungsgemäßen Verbindungen bzw. Salzen insbesondere die Aromastoffe aus der nachfolgend angegebenen Liste bevorzugter Aromastoffe besonders geeignet: Diacetyl, Acetoin, Benzaldehyd, Furaneol, Heliotropin, Vanillin, Ethylvanillin, Ethylvanillinisobutyrat (= 3-Ethoxy-4-isobutyryloxybenzaldehyd), Furaneol® (2,5-Dimethyl-4-hydroxy-3(2H)-furanon) und dessen Derivate (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Derivate (dabei vorzugsweise Ethylmaltol), Cumarin und Cumarin-Derivate, gamma-Lactone (dabei vorzugsweise gamma-Undecalacton, gamma-Nonalacton, gamma-Decalalcton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methylbuttersäureethylester, n-Hexansäureethylester, n-Hexansäureallytester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd.

Besonders bevorzugt sind erfindungsgemäße Zubereitungen, in denen die Gesamtmenge an eingesetzten Aromastoffen, vorzugsweise an eingesetzten Aromastoffen aus der vorstehend angegebenen Liste bevorzugter Aromastoffe, so gewählt ist, dass sie die Wirkung der eingesetzten erfindungsgemäßen Verbindung bzw. des eingesetzten erfindungsgemäßen Salzes bzw. einer entsprechenden Mischung, einen unangenehmen Geschmackseindruck zu maskieren oder zumindest zu vermindern, in synergistischer Weise verstärkt. Insbesondere bei Anwesenheit von Aromastoffen, welche die geschmacksmaskierenden oder ―vermindernden Eigenschaften der erfindungsgemäßen Verbindungen bzw. Salze synergistisch verstärken, kann die Gesamtmenge der eingesetzten erfindungsgemäßen Verbindungen und erfindungsgemäßen Salze sehr gering gewählt werden und dennoch eine vollständige Maskierung oder zumindest eine signifikante Verminderung der unangenehmen Geschmackseindrücke erreicht werden. Hinsichtlich der bevorzugten Auswahl der einzusetzenden erfindungsgemäßen Verbindungen bzw. Salze sowie hinsichtlich der bevorzugten Aromastoffe gilt das jeweils weiter oben Gesagte entsprechend.

In eigenen Untersuchungen trat eine solche Verstärkung insbesondere auf, wenn das Gewichtsverhältnis der Gesamtmengen von erfindungsgemäßen Verbindungen und erfindungsgemäßen Salzen einerseits und der Gesamtmenge an Aromastoffen aus der vorstehend angegebenen Liste bevorzugter Aromastoffe andererseits im Bereich von 1:2 bis 1:200 lag, vorzugsweise im Bereich von 1:3 bis 1:100, besonders bevorzugt im Bereich von 1:5 bis 1:70. Entsprechende Zubereitungen sind erfindungsgemäß bevorzugt.

Die erfindungsgemäßen Verbindungen und erfindungsgemäßen Salze sind besonders gut dazu geeignet, den unangenehmen Geschmackseindruck bestimmter unangenehm schmeckender Stoffe zu maskieren bzw. zu vermindern. Zu diesen unangenehm schmeckenden Stoffen gehören insbesondere die folgenden:
Xanthinalkaloide, Xanthine (Coffein, Theobromin, Theophyllin und Methylxanthine), Alkaloide (Chinin, Brucin, Strychnin, Nicotin), phenolische Glycoside (z.B. Salicin, Arbutin), Flavonoidglycoside (z.B. Neohespereidin, Hesperidin, Naringin, Quercitrin, Rutin, Hyperosid, Quercetin-3-O-glucosid, Myricetin-3-O-glycoside), Chalcone oder Chalconglycoside (z.B. Phloridzin, Phloridzinxyloside), hydrolisierbare Tannine (Gallus- oder Elagsäureester von Kohlenhydraten, z.B. Pentagalloylglucose, Tanninsäuren), nichthydrolisierbare Tannine (ggfs. galloylierte Catechine, Gallocatechine, Epigallocatechine oder Epicatechine und deren Oligomeren, z.B. Proanthyocyanidine oder Procyanidine, Thearubigenin), Flavone (z.B. Quercetin, Taxifolin, Myricetin), Phenole wie z.B. Salicin, Polyphenole (z.B. gamma-Oryzanol, Kaffeesäure oder deren Ester (z. B. Chlorogensäure und Isomere)), terpenoide Bitter- und Gerbstoffe (z.B. Limonoide wie Limonin oder Nomilin aus Zitrusfrüchten, Lupolone und Humolone aus Hopfen, Iridoide, Secoiridoide), Absinthin aus Wermut, Amarogentin aus Enzian, metallische Salze (insbesondere Kalium-, Magnesium- und Calciumsalze, Kaliumchlorid, Kaliumgluconat, Kaliumcarbonat, Kaliumsulfat, Kaliumlactat, Kaliumglutamat, Kaliumsuccinat, Kaliummalat, Natriumsulfat, Magnesiumsulfat, Aluminiumsalze, Zinksalze, Zinnsalze, Eisen-(II)-salze, Eisen-(III)-salze, Chrom-(II)-picolinat), pharmazeutische Wirkstoffe (z.B. Fluorchinolon-Antibiotika, Paracetamol, Aspirin, beta--Lactam-Antibiotika, Ambroxol, Propylthiouracil [PROP], Guaifenesin), Vitamine (beispielsweise Vitamin H, Vitamine aus der B-Reihe wie Vitamin B1, B2, B6, B12, Niacin, Panthotensäure), Denatoniumbenzoat, Sucraloseoctaacetat, Eisensalze, Aluminiumsalze, Zinksalze, Harnstoff, ungesättigte Fettsäuren, insbesondere ungesättigte Fettsäuren in Emulsionen, bitter/adstringierend schmeckende Aminosäuren (z.B. Leucin, Isoleucin, Valin, Tryptophan, Prolin, Histidin, Tyrosin, Lysin oder Phenylalanin) und bitter oder adstringierend schmeckende Peptide oder Proteine (insbesondere Peptide mit einer Aminosäure aus der Gruppe Leucin, Isoleucin, Valin, Tryptophan, Prolin oder Phenylalanin am N- oder C-Terminus), Saponine, insbes. Sojasaponine, Isoflavonoide (insbes. Genistein, Daidzein, Genistin, Daidzin, deren Glycoside und acylierten Glycoside).

Es versteht sich, dass bevorzugte erfindungsgemäße Zubereitungen neben einer erfindungsgemäßen Verbindung, einem erfindungsgemäßen Salz oder einer entsprechenden Mischung auch ein oder mehrere unangenehm schmeckende Stoffe umfassen, die aus der vorstehenden Liste ausgewählt sind.

In einer erfindungsgemäßen Zubereitung kann eine erfindungsgemäße Verbindung bzw. ein erfindungsgemäßes Salz auch mit Stoffen kombiniert sein, die einen nicht unangenehmen Primärgeschmack (beispielsweise süß, salzig, würzig, sauer) und/oder ―geruch besitzen, aber dennoch einen unangenehmen Geschmackseindruck vermitteln, insbesondere also einen adstringierenden, bitteren, trockenen, staubigen, mehligen, kalkigen und/oder metallischen Geschmackseindruck (insbesondere Nachgeschmack oder Beinote/Nebennote). Zu solchen Stoffen mit einem nicht unangenehmen Primärgeschmack und/oder ―geruch einerseits, aber einer unangenehmen geschmacklichen Beinote oder einem unangenehmen Nachgeschmack andererseits gehören zum Beispiel bestimmte Süßstoffe, Zuckeraustauschstoffe und Aromastoffe. Beispielsweise seien genannt: Kaliumsalze (insbesondere Kaliumchlorid, Kaliumgluconat, Kaliumcarbonat, Kaliumsulfat, Kaliumlactat, Kaliumglutamat, Kaliumsuccinat, Kaliummalat), Aspartam, Acesulfam K, Neotam, Superaspartam, Saccharin, Sucralose, Tagatose, Monellin, Stevioside, Rebaudioside, Hernandulcin, Thaumatin, Miraculin, Glycyrrhizin, Glycyrrhetinsäure oder deren Derivate, Cyclamat oder die pharmazeutisch akzeptablen Salze der vorgenannten Verbindungen.

Es versteht sich, dass derartige Stoffe mit einem nicht unangenehmen Primäreindruck, aber einem unangenehmen Nebengeschmack bzw. Nachgeschmack bevorzugt in erfindungsgemäßen Zubereitungen vorgesehen sind.

Selbstverständlich sind sämtliche vorstehend im Zusammenhang mit erfindungsgemäßen Zubereitungen erfolgten Erläuterungen ebenso zutreffend für die erfindungsgemäßen Verwendungen, und umgekehrt.

Wie bereits weiter oben erwähnt, ist es besonders überraschend, dass bei Verwendung einer Gesamtmenge erfindungsgemäßer Verbindungen bzw. Salze, die (noch) nicht ausreicht, den Speichelfluss zu verstärken, dennoch die unangenehmen Geschmackseindrücke eines unangenehm schmeckenden Stoffes (wie weiter oben definiert) maskiert oder zumindest vermindert werden. Dementsprechend sind erfindungsgemäße Zubereitungen bevorzugt, bei denen die in der Zubereitung enthaltene Gesamtmenge an dem oder den erfindungsgemäßen Verbindungen bzw. Salzen nicht ausreicht, den Speichelfluss zu verstärken. Insbesondere die vorstehend als bevorzugt genannten erfindungsgemäßen Verbindungen bzw. Salze sind in der Lage, den unangenehmen Geschmackseindruck unangenehm schmeckender Stoffe zu maskieren oder zumindest zu vermindern, ohne dabei einen chemesthetischen Reiz zu vermitteln und/oder den Speichelfluss zu verstärken.

Erfindungsgemäße Zubereitungen liegen vorzugsweise als Halbfertigware, als Riech-Aroma- oder Geschmacksstoffkomposition oder als Würzmischung vor.

Es wurde weiter oben hervorgehoben, dass die erfindungsgemäßen Verbindungen und Salze vorteilhafterweise als Geschmacksmaskierer eingesetzt werden können, wobei sie selbst in den üblichen Einsatzkonzentrationen keinen signifikanten chemesthetischen Reiz vermitteln. Dementsprechend sind auch erfindungsgemäße Zubereitungen vielfach bevorzugt, die insgesamt keinen chemesthetischen Geschmackseindruck vermitteln. Für bestimmte Einsatzzwecke ist es jedoch sinnvoll oder erforderlich, in einer erfindungsgemäßen Zubereitung neben einer oder mehreren erfindungsgemäßen Verbindungen bzw. Salzen zumindest eine weitere Substanz zum Maskieren oder Vermindern des unangenehmen Geschmackseindrucks eines unangenehm schmeckenden Stoffes oder Stoffgemisches vorzusehen. Eine solche weitere Substanz (ein weiterer Geschmacksmaskierer) wird dann im Einzelfall durchaus einen chemesthetischen Reiz hervorrufen, der aber in der Einzelzubereitung gewünscht ist. In solchen bevorzugten erfindungsgemäßen Zubereitungen liegt dann eine Kombination von zwei oder mehr Geschmackskorrigenzien vor, wobei zumindest eine Geschmackskorrigenz eine erfindungsgemäße Verbindung oder ein erfindungsgemäßes Salz ist und zumindest eine weitere Geschmackskorrigenz eine Substanz ist, bei der es sich nicht um eine erfindungsgemäße Verbindung oder ein erfindungsgemäßes Salz handelt.

Die erfindungsgemäßen Verbindungen und erfindungsgemäßen Salze sind insbesondere zur Kombination mit den Alkamiden geeignet, die in der Veröffentlichung US 2009 0124 701 A1 als Geschmacksmaskierer offenbart sind. Dementsprechend sind erfindungsgemäße Zubereitungen bevorzugt, in denen ein oder mehrere Verbindungen enthalten sind, die ausgewählt sind aus der Gruppe bestehend aus 2E,4E-Decadiensäure-N-isobutylamid (*trans*-Pellitorin); 2E,4Z-Decadiensäure-N-isobutylamid (*cis*-Pellitorin); 2Z,4Z-Decadiensäure-N-isobutylamid; 2Z,4E-Decadiensäure-N-isobutylamid; 2E,4E-Decadiensäure-N-([2S]-2-methylbutyl)amid; 2E,4E-Decadiensäure-N-([2S]-2-methylbutyl)amid; 2E,4E-Decadiensäure-N-([2R]-2-methylbutylamid); 2E,4Z-Decadiensäure-N-(2-methylbutyl)amid; 2E,4E-Decadiensäure-N-piperid (Achilleamid); 2E,4E-Decadiensäure-N-piperid (Sarmentin); 2E-Decensäure-N-isobutylamid; 3E-Decensäure-N-isobutylamid; 3E-Nonensäure-N-isobutylamid; 2E,6Z,8E-Decatriensäure-N-isobutylamid (Spilanthol); 2E,6Z,8E-Decatriensäure-N-([2S]-2-methylbutyl)amid (Homospilanthol); 2E,6Z,8E-Decatriensäure-N-([2R]-2-methylbutyl)amid; 2E-Decen-4-insäure-N-isobutylamid; 2Z-Decen-4-insäure-N-isobutylamid; 2E,6Z,8E,10E-Dodecatetraensäure-N-(2-methylpropyl)amid (alpha-Sanshool); 2E,6Z,8E,10E-Dodecatetraensäure-N-(2-hydroxy-2-methylpropyl)amid (alpha-Hydroxysanshool); 2E,6E,8E,10E-Dodecatetraensäure-N-(2-hydroxy-2-methylpropyl)amid (gamma-Hydroxysanshool); 2E,4E,8Z,10E,12E-Tetradecapentaensäure-N-(2-hydroxy-2-methylpropyl)amid (gamma-Hydroxysanshool); 2E,4E,8E,10E,12E-Tetradecapentaensäure-N-(2-hydroxy-2-methylpropyl)amid (gamma-Hydroxyisosanshool); 2E,4E,8Z,10E,12E-Tetradecapentaensäure-N-(2-methyl-2-propenyl)amid (gamma-Dehydrosanshool); 2E,4E,8Z,10E,12E-Tetradecapentaensäure-N-(2-methylpropyl)amid (gamma-Sanshool); 2E,4E,8Z,11Z-Tetradecatetraensäure-N-(2-hydroxy-2-methylpropyl)amid (Bungeanool); 2E,4E,8Z,11E-Tetradecatetraensäure-N-(2-hydroxy-2-methylpropyl)amid (Isobungeanool); 2E,4E,8Z-Tetradecatriensäure-N-(2-hydroxy-2-methylpropyl)amid (Dihydrobungeanool) und 2E,4E-Tetradecadiensäure-N-(2-hydroxy-2-methylpropyl)amid (Tetrahydrobungeanool).

In eigenen Untersuchungen hat es sich überraschenderweise herausgestellt, dass eine Gesamtkonzentration an erfindungsgemäßen Verbindungen und Salzen von unter 100 ppm, bevorzugt unter 75 ppm, besonders bevorzugt unter 50 ppm, und wenigstens 0,1 ppm, bevorzugt wenigstens 1 ppm (bezogen auf das Gesamtgewicht der Zubereitung) ausreicht, um in der Zubereitung den unangenehmen Geschmackseindruck, insbesondere den adstringierenden Geschmackseindruck einer Vielzahl von Stoffen zu maskieren oder zumindest zu vermindern, wenn diese in üblichen Konzentrationen vorliegen. Insbesondere maskieren oder vermindern die erfindungsgemäßen Verbindungen und Salze in den genannten Gesamtkonzentrationen den Geschmackseindruck einer Vielzahl von Stoffen in bestimmten Anwendungen, insbesondere den unangenehmen Geschmackseindruck von Quercitrin, Rutin, Phloridzin, Gallus- oder Elagsäureester von Kohlenhydraten (z.B. Pentagalloylglucose), ggfs. galloylierten Catechinen, Gallocatechinen, Epigallocatechinen oder Epicatechinen, Proanthyocyanidinen oder Procyanidinen, Thearubigenin, Quercetin, Taxifolin, Myricetin, gamma-Oryzanol, Kaffeesäure oder deren Ester (z.B. Chlorogensäure und Isomere), metallischen Salzen (insbesondere Aluminiumsalze, Zinksalze, Eisen-(II)-salze), adstringierend schmeckenden Peptiden oder Proteinen (insbesondere Peptide mit einer Aminosäure aus der Gruppe Leucin, Isoleucin, Valin, Tryptophan, Prolin oder Phenylalanin am N- oder C-Terminus), Saponinen, insbes. Sojasaponinen und Isoflavonoiden (insbes. Genistein, Daidzein, Genistin, Daidzin, deren Glycoside und acylierten Glycoside), künstlichen Süßstoffen oder Zuckeraustauschstoffen (insbes. Aspartam, Acesulfam K, Saccharin, Sucralose, Tagatose, Cyclamat u.a.) in einer Vielzahl von Anwendungen wie z.B. Kakao-haltigen Produkten, Schwarz- oder Grüntee-haltigen Produkten, Traubenkernextrakt-haltigen Produkten, Rotwein enthaltenden Produkten, fettreduzierten oder fettfreien Milchprodukten wie Joghurt oder Käse, fettreduzierten Fettprodukten auf Emulsionsbasis wie Mayonnaise, Margarine, auf Soja basierenden Anwendungen (z.B. Sojamilch, Sojaproteinhaltigen Getränken, Soja-Eis etc., Tofu), kalorienreduzierten Produkten mit künstlichen Süßstoffen und Zuckeraustauschstoffen (z.B. Getränke, Milchprodukte, Süßwaren, etc.).

Hierbei ist es selbstverständlich wieder besonders vorteilhaft, dass die erfindungsgemäßen Verbindungen und Salze in den vorstehend genannten sehr geringen Konzentrationen nahezu keinen Eigengeschmack besitzen und insbesondere keinen signifikanten chemesthetischen Reiz vermitteln, insbesondere keinen kribbelnden oder scharfen oder betäubenden sensorischen Eindruck. Sie beeinflussen somit die weiteren, in der Regel nicht unangenehmen Geschmacksqualitäten der genannten Substanzen und etwaiger weiterer anwesender Aromastoffe nicht negativ; insbesondere beeinflussen sie den süßen Geschmack von süßen Stoffen nicht negativ, sondern vielmehr sogar positiv. Durch die Eigenschaften der erfindungsgemäßen Verbindungen und Salze wird das Mundgefühl in den vorstehend genannten Anwendungen wesentlich verbessert.

Eine besonders bevorzugte erfindungsgemäße Zubereitung ist somit eine, die bezogen auf ihr Gesamtgewicht, eine Gesamtmenge an erfindungsgemäßen Verbindungen und Salzen im Bereich von 0,1 ppm, bevorzugt 1 ppm, bis 100 ppm, bevorzugt 75 ppm, besonders bevorzugt 50 ppm, umfasst.

Besonders bevorzugt sind Zubereitungen, bei denen die Gesamtmenge an vorstehend als bevorzugt bezeichneten Verbindungen im angegebenen Bereich liegt.

Erfindungsgemäße pharmazeutische Zubereitungen umfassen einen pharmazeutischen Wirkstoff. Im Rahmen der vorliegenden Erfindung vorteilhafte pharmazeutische Wirkstoffe sind beispielsweise steroidale entzündungshemmende Substanzen vom Kortikosteroiden-Typ wie z.B. Hydrocortison, Hydrocortison-Derivate wie Hydrocortison-17-butyrat, Dexamethason, Dexamethasonphosphat, Methylprednisolon oder Cortison.

Im Rahmen der vorliegenden Erfindung vorteilhafte nichtsteroidale pharmazeutische Wirkstoffe sind beispielsweise Entzündungshemmer wie Oxicame wie Piroxicam oder Tenoxicam; Salicylate wie Aspirin® (Acetylsalicylsäure), Disalcid, Solprin oder Fendosal; Essigsäure-Derivate wie Diclofenac, Fenclofenac, Indomethacin, Sulindac, Tolmetin, oder Clindanac; Fenamate wie Mefenamic, Meclofenamic, Flufenamic oder Niflumic; Propionsäure-Derivate wie Ibuprofen, Naproxen, Flurbiprofen, Benoxaprofen oder Pyrazole wie Phenylbutazon, Oxyphenylbutazon, Febrazon oder Azapropazon.

Besonders bevorzugte erfindungsgemäße pharmazeutische Zubereitungen enthalten Wirkstoffe ausgewählt aus der Gruppe bestehend aus Paracetamol, Acetylsalicylsäure oder Ibuprofen, Vitaminen (beispielsweise Vitamin H, Vitamine aus der B-Reihe wie Vitamin B1, B2, B6, B12, Niacin, Panthotensäure, vorzugsweise in Form von (Brause-)Tabletten oder Kapseln), Mineralien (vorzugsweise in Form von (Brause-)Tabletten oder Kapseln) wie Eisensalze, Zinksalze, Selensalze, Wirkstoffe und Extrakte aus Spitzwegerich (z.B. in Hustensirup) und Johanniskraut. Solche besonders bevorzugten Arzneimittel sind vorzugsweise nicht verschreibungspflichtige Produkte oder freiverkäufliche Arzneimittel, d.h. sogenannte OTC ("over the counter")-Präparate.

Der Ernährung oder dem Genuss dienende erfindungsgemäße Zubereitungen können insbesondere sein: Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nichtalkoholische Getränke (z.B. Kakao, Kaffee, Grüntee, Schwarztee, mit (Grün-, Schwarz-)Tee-Extrakten angereicherte (Grün-, Schwarz-)Tee-Getränke, Rooibos-Tee, andere Kräutertees, Weine, weinhaltige Getränke, Biere, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Vollfett- oder fettreduzierte oder fettfreie Milchgetränke, Milchreis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Getränke, enthaltend isoliertes oder enzymatisch behandeltes Sojaprotein, Sojamehl enthaltende Getränke, Sojalecithin-haltige Zubereitungen, fermentierte Produkte wie Tofu oder Tempe oder daraus gefertigte Produkte und Mischungen mit Fruchtzubereitungen und fakultativ Aromen), Fruchtzubereitungen (z.B. Konfitüren, Fruchteis, Fruchtsoßen, Fruchtfülfungen), Gemüsezubereitungen (z.B. Ketchup, Soßen, Trockengemüse, Tiefkühlgemüse, vorgegartes Gemüse, eingekochtes Gemüse), Knabberartikel (z.B. gebackene oder frittierte Kartoffelchips oder Kartoffelteigprodukte, Extrudate auf Mais- oder Erdnussbasis), Produkte auf Fett- und Ölbasis oder Emulsionen derselben (z.B. Mayonnaise, Remoulade, Dressings, jeweils Vollfett- oder fettreduziert), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden, Süßstoffzubereitungen, -tabletten oder -sachets, sonstige Zubereitungen zum Süßen oder Weißen von Getränken oder anderen Nahrungsmitteln. Die Zubereitungen im Sinne der Erfindung können auch als Halbfertigware zur Herstellung weiterer der Ernährung oder dem Genuss dienenden Zubereitungen dienen. Die Zubereitungen im Sinne der Erfindung können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen und als Nahrungsergänzungsmittel vorliegen.

Besonders bevorzugt sind erfindungsgemäße Zubereitungen, welche in Abwesenheit einer den unangenehmen Geschmack maskierenden bzw. vermindernden Gesamtmenge an erfindungsgemäßen Verbindungen und Salzen adstringierend schmecken würden. Solche Zubereitungen sind vorzugsweise ausgewählt aus der Gruppe bestehend aus Kakao-haltigen Produkten, Grüntee-bzw. Schwarzteezubereitungen, mit auf *Camellia ssp*. basierten (Weiß-, Gelb-, Grün-, Rot-, Oolong-, Schwarz-) Tee-Extrakten angereicherten (Grün-, Schwarz-) Tee-Getränken, Mate-Tee, Rotbusch-Tee, andere Kräutertees, Weinen, weinhaltigen Getränken, Instantgetränken (z.B. Instant-Kakao-Getränke, Instant-Tee-Getränke, Instant-Kaffeegetränke), fettreduzierten oder fettfreien Milchgetränken, Milchreis, Joghurt, Kefir, Frischkäse, Weichkäse, Hartkäse, Trockenmilchpulver, Molke, Butter, Buttermilch, teilweise oder ganz hydrolisierten Milchprotein-haltigen Produkten, Produkten aus Sojaprotein oder anderen Sojabohnen-Fraktionen (z.B. Sojamilch und daraus gefertigte Produkte, Getränke, enthaltend isoliertes oder enzymatisch behandeltes Sojaprotein, Sojamehl enthaltende Getränke, Sojalecithin-haltige Zubereitungen, fermentierte Produkte wie Tofu oder Tempe oder daraus gefertigte Produkte und Mischungen mit Fruchtzubereitungen und fakultativ Aromen), fettreduzierten Produkten auf Fett- und Ölbasis oder Emulsionen derselben (z.B. Mayonnaise, Remoulade, Dressings), zusätzlich Süßstoffe enthaltenden Produkten wie kalorienreduzierte oder ―freie Getränke, vor allem alkoholfreien Erfrischungsgetränken, Instantgetränken, zuckerfreien oder - armen Eiscremes, Milchprodukten, Backwaren, Konfekt, Schokolade, Sojamilch- oder - protein-haltigen Produkten.

Wie bereits des Öfteren erwähnt, sind die oben definierten Zubereitungen gemäß einem Aspekt der vorliegenden Erfindung insbesondere (i) auf Soja basierende Produkte und (ii) fettreduzierte (low-fat) Joghurts wie oben definiert mit adstringierendem Geschmacks, In diesen Produkten ist der erfindungsgemäße Maskierungserfolg besonders ausgeprägt.

Der Mundpflege dienende erfindungsgemäße Zubereitungen sind insbesondere Mund- und/oder Zahnpflegemittel wie Zahnpasten, Zahngele, Zahnpulver, Mundwässer, Kaugummis und andere Mundpflegemittel.

Erfindungsgemäße Zahnpflegemittel (als Beispiel für erfindungsgemäße Zubereitungen, welche der Mundpflege dienen) umfassen neben den erfindungsgemäßen Verbindungen bzw. Salzen regelmäßig weitere Substanzen bzw. Materialien die ausgewählt sind aus der Gruppe bestehend aus: abrasiven Systemen (Schleif- oder Poliermittel), wie z.B. Kieselsäuren, Calciumcarbonate, Calciumphosphate, Alumiuniumoxide und/oder Hydroxylapatite; oberflächenaktiven Substanzen wie z.B. Natriumlaurylsulfat, Natriumlaurylsarcosinat und/oder Cocamidopropylbetain; Feuchthaltemitteln wie z.B. Glycerin und/oder Sorbit; Verdickungsmitteln, wie z.B. Carboxymethylcellulose, Polyethylenglycole, Carrageenan und/oder Laponite^{®}; Süßstoffen, wie z.B. Saccharin; anderen Geschmackskorrigenzien für unangenehme Geschmackseindrücke; Geschmackskorrigenzien für weitere, in der Regel nicht unangenehme Geschmackseindrücke; geschmacksmodulierenden Stoffen (z.B. Inositolphosphat, Nucleotide wie Guanosinmonophosphat, Adenosinmonophosphat oder andere Stoffe wie Natriumglutamat oder 2-Phenoxypropionsäure); Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid); Icilin-Derivaten; Stabilisatoren und aktiven Wirkstoffen, wie z.B. Natriumfluorid, Natriummonofluorphosphat, Zinndifluorid, quartäre Ammoniumfluoride, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid; Aromen; Natriumbicarbonat; Geruchskorrigenzien.

Kaugummis (als weiteres Beispiel für der Mundpflege dienende erfindungsgemäße Zubereitungen) umfassen regelmäßig eine Kaugummibase, d.h. eine beim Kauen plastisch werdende Kaumasse. Darüberhinaus sind in erfindungsgemäßen Kaugummis vorzugsweise Substanzen und Materialien enthalten, die aus der Liste ausgewählt sind bestehend aus: Zuckern (verschiedener Arten); Zuckeraustauschstoffen; Süßstoffen; Zuckeralkoholen; weiteren Geschmackskorrigenzien für unangenehme Geschmackseindrücke; Geschmackskorrigenzien für weitere, in der Regel nicht unangenehme Geschmackseindrücke; geschmacksmodulierenden Stoffen (z.B. Inositolphosphat, Nucleotide wie Guanosinmonophosphat, Adenosinmonophosphat oder andere Stoffe wie Natriumglutamat oder 2-Phenoxypropionsäure); Kühlwirkstoffen (siehe dazu den vorangehenden Absatz); Feuchthaltemitteln (siehe dazu den vorangehenden Absatz); Verdickern (siehe dazu den vorangehenden Absatz); Emulgatoren; Aromen (vgl. dazu die entsprechenden Ausführungen weiter oben); Stabilisatoren; Geruchskorrigenzien.

Die vorliegende Erfindung betrifft auch ein Verfahren zum Maskieren oder Vermindern des unangenehmen Geschmackseindrucks eines unangenehm schmeckenden Stoffes oder Stoffgemisches, mit folgendem Schritt: Vermischen eines unangenehm schmeckenden Stoffes oder Stoffgemisches mit
- einer erfindungsgemäßen Verbindung oder einem erfindungsgemäßen Salz (vorzugsweise wie vorstehend als bevorzugt bezeichnet)
   oder
- einer Mischung umfassend oder bestehend aus zwei oder mehr unterschiedlichen erfindungsgemäßen Verbindungen oder Salzen (wie vorstehend definiert; vorzugsweise wie vorstehend als bevorzugt bezeichnet).

Für die Auswahl von erfindungsgemäßen Verbindungen bzw. Salzen sowie für die Auswahl der unangenehm schmeckenden Stoffe in einem erfindungsgemäßen Verfahren gilt das weiter oben mit Blick auf die erfindungsgemäßen Verbindungen, Salze, Mischungen, Zubereitungen und Verwendungen Gesagte entsprechend.

Bevorzugte erfindungsgemäße Verfahren sind somit solche, in denen der unangenehme Geschmackseindruck ausgewählt ist aus der Gruppe bestehend aus adstringierend, bitter, trocken, staubig, mehlig, kalkig und metallisch.

Erfindungsgemäße Zubereitungen, die erfindungsgemäße Verbindungen und/oder erfindungsgemäße Salze umfassen, werden vorzugsweise hergestellt, indem die jeweilige bzw. die jeweiligen Verbindungen und/oder Salze als Substanzen, als Lösung oder in Form eines Aromagemisches in eine der Ernährung, der Mundpflege oder dem Genuss dienende oder orale, pharmazeutische Basis-Zubereitung eingearbeitet werden. Vorteilhafterweise können als Lösung vorliegende erfindungsgemäße Zubereitungen auch z.B. durch Sprühtrocknung in eine feste erfindungsgemäße Zubereitung überführt werden.

Gemäß einer weiteren bevorzugten Ausführungsform werden zur Herstellung erfindungsgemäßer Zubereitungen die erfindungsgemäßen Verbindungen und/oder erfindungsgemäßen Salze und gegebenenfalls weitere Bestandteile der erfindungsgemäßen Zubereitung in Form von Emulsionen, in Liposomen, z.B. ausgehend von Phosphatidylcholin, in Microsphären, in Nanosphären oder auch in Kapseln, Granulaten oder Extrudaten aus einer für Lebens- und Genussmittel geeigneten Matrix, z.B. aus Stärke, Stärkederivaten, Cellulose oder Cellulosederivaten (z.B. Hydroxypropylcellulose), anderen Polysacchariden (z.B. Alginat), natürlichen Fetten, natürlichen Wachsen (z.B. Bienenwachs, Carnaubawachs) oder aus Proteinen, z.B. Gelatine, eingearbeitet. In einem bevorzugten Herstellungsverfahren werden die erfindungsgemäßen Verbindungen und/oder erfindungsgemäßen Salze vor dem Einarbeiten mit einem oder mehreren geeigneten Komplexbildnern, beispielsweise mit Cycloglycanen, z.B. cyclofructanen, Cyclodextrinen oder Cyclodextrinderivaten, bevorzugt alpha-, beta- und gamma-Cyclodextrin, komplexiert und in dieser komplexierten Form eingesetzt.

Besonders bevorzugt ist eine erfindungsgemäße Zubereitung, bei der die Matrix so gewählt wird, dass die erfindungsgemäßen Verbindungen und/oder erfindungsgemäßen Salze verzögert von der Matrix freigegeben werden, so dass man eine langanhaltende Wirkung erhält.

### Beispiele

Die Beispiele dienen zur Verdeutlichung der Erfindung. Prozentangaben beziehen sich - wie auch ansonsten im gesamten Text - auf das Gesamtgewicht, sofern nicht anders angegeben.

### Synthese-Beispiel: Herstellung von (2S)-2-[(2E,4E)-Deca-2,4-dienoylamino]propionsäure (Verbindung 4) via (2S)-2-[(2E,4E)-deca-2,4-dienoylamino]propionsäuremethylester (Verbindung 3)

### a) 2S)-2-[(2E,4E)-deca-2,4-dienoylamino]propionsäuremethylester (Verbindung 3):

25.0 g (0.18 mol) L-Alaninmethylester Hydrochlorid werden in 500 ml Dichlormethan vorgelegt und bei 10 bis 15°C mit 24.0 g (0.30 mol) Pyridin versetzt. Anschließend werden bei derselben Temperatur 37.0 g (0.20 mol) 2E,4E-Decadiensäurechlorid zugegeben und für weitere 12 Stunden bei Raumtemperatur gerührt. Anschließend wird die Reaktionsmischung dreimal mit 5%iger wässriger Salzsäure, einmal mit gesättigter wässriger Natriumhydrogencarbonatlösung sowie zweimal mit Wasser gewaschen. Nach Trocknen über Natriumsulfat und Entfernen des Lösungsmittels am Rotationsverdampfer wird der Feststoff in 50 ml MTBE (Methyl-tert-butylether) gelöst und bei - 20 °C wieder ausgefällt. Der erstandene Kristallbrei wird in 100 ml Hexan suspendiert. Nach Filtration wird zur weiteren Aufreinigung aus 150 ml Hexan/MTBE (2:1) umkristallisiert. Es werden 24.1 g (0.095 mol) des erwarteten Produkts erhalten.

### b) (2S)-2-[(2E,4E)-deca-2,4-dienoylamino]propionsäure (Verbindung 4):

22.5 g (0.089 mol) des unter a) hergestellten Methylesters werden zu einer Lösung aus 3.9 g (0.098 mol) Natriumhydroxid in 160 ml Wasser gegeben. Die Reaktionsmischung wird für 48 Stunden bei Raumtemperatur gerührt und anschleißend mit 500 ml Wasser verdünnt und mit 50.0 g 10%iger wässriger Salzsäure versetzt. Der ausgefallene Feststoff wird abfiltriert und getrocknet. Zur weiteren Aufreinigung wird aus 170 ml MTBE umkristallisiert.

Ausbeute: 11.6 g (0.049 mol)

### Analysendaten:

**¹H-NMR (400 MHz, CDCl₃).** 0.89 (t, *J* = 6.9 Hz; 3H); 1.25 ― 1.46 (kB, 6H); 1.49 (s, *J* = 7.2 Hz, 3H); 2.15 (m, 2H); 4.64 (dq, *J* = 7.0/7.2 Hz, 1H); 5.82 (d, *J* = 15.0 Hz, 1 H); 6.10 ― 6.15 (kB, 2H); 6.75 (d, *J* = 7.0 Hz, 1 H); 7.22 (m, 1 H) 7.79 (bs, 1 H) ppm.

**¹³C-NMR (100 MHz, CDCl₃):** 14.0 (CH₃); 18.0 (CH₃); 22.5 (CH₂); 28.4 (CH₂); 31.4 (CH₂); 33.0 (CH₂); 48.6 (CH); 120.3 (CH); 128.1 (CH); 143.1 (CH); 144.8 (CH); 167.2 (C=O); 175.7 (C=O) ppm.

**HRMS** [C₁₃H₂₁NO₃]: ber.: 239,1521 gef.: 239.1515

### Anwendungsbeispiel 1: Untersuchung der geschmacklichen Eigenschaften der Verbindungen (1) bis (6) (sensorische Profilierung)

Die Verbindungen (1) bis (6) wurden in einer 5 %igen wässrigen Saccharoselösung bzw. einer 0,5 %igen wässrigen Kochsalzlösung jeweils in einer Konzentration von 50 ppm gelöst und anschließend durch ein Expertenpanel (4 bis 6 Personen, davon 2 bis 3 Flavoristen) auf ihre generellen geschmacklichen Eigenschaften untersucht.

### Ergebnis der Bewertung:

- Verbindung (1):: sehr schwache Getreide-Note, mundwässernd, sonst neutral
- Verbindung (2):: sehr schwache Getreide-Note, mundwässernd, kaum wahrnehmbar tingling, sonst neutral
- Verbindung (3):: sehr schwache Getreide-Note, mundwässernd, in Salzlösung schwach bitter, sonst neutral
- Verbindung (4):: sehr schwache Getreide-Note, saftig, mundwässernd, kaum wahrnehmbar tingling
- Verbindung (5):: sehr schwache grüne Note, kaum wahrnehmbar tingling, sonst neutral
- Verbindung (6):: leichte Birnen-Note, sonst neutral

### Anwendungsbeispiel 2: Zeitabhängige Verminderung der Adstringenz und Bitterkeit von Epigallocatechingallat (EGCG)

Um verschiedene Attribute (Deskriptoren), wie z. B. Adstringenz und Bitterkeit einer Probe gleichzeitig über einen definierten Zeitraum zu bewerten, wurde die Methode des "Multiple Time-Intensity Profiling" (mTIP) angewandt. Diese deskriptive Methode vereinigt die Vorteile der "Dual-Attribute Time-Intensity" (DATI) -Methode, bei der die Intensitäten von 2 Deskriptoren simultan bewertet werden können, sowie der "Time-Intensity Profiling" (TIP) -Methode, bei der die Intensität eines Deskriptors als Funktion der Zeit evaluiert wird.

Bei jedem mTIP-Test werden trainierte Panelisten (n=15) gebeten, nacheinander 2 Schlucke (je 5 ml) der zu bewertenden Probe in den Mund zu nehmen, den kompletten Mundraum damit zu benetzen und die Probe danach wieder auszuspucken. Während des Tests werden die Intensitäten der verschiedenen Deskriptoren auf horizontalen Linienskalen, die auf dem Computerbildschirm abgebildet sind, markiert. Für jeden Deskriptor ist jeweils eine Linienskala abgebildet. Somit ist eine parallele Bewertung mehrerer Attribute möglich. Die Intensitäten werden sofort zunächst unmittelbar nach Ausspucken der Probe und dann zu den in den Tabellen 1 und 2 genannten Zeitpunkten nach dem Ausspucken über einen Zeitraum von 70 Sekunden von den Panelisten bewertet. Für jeden Bewertungszeitpunkt steht den Panelisten dabei ein Zeitfenster von ca. 20 Sekunden zur Verfügung, um die verschiedenen Deskriptoren genau zu bewerten; dieses Zeitfenster endet jeweils zu den genannten Zeitpunkten. Die dabei entstehenden Daten werden mittels Compusense^{®} *five* (software-Paket; siehe hierzu http://www.compusense.com/software/five/) analysiert und grafisch in einer Zeit-Intensitäts-Kurve dargestellt.

Um den bitter- und adstringenz-maskierenden Effekt von (2*S*)-2-[(2*E*,4*E*)-Deca-2,4-dienoylamino]propionsäure (Verbindung 4) aufzuzeigen, wurde das Panel unter anderem mit einer wässrigen (nicht erfindungsgemäßen) Testlösung geschult, die 750 ppm Epigallocathechingallat (EGCG) und 125 ppm Ascorbinsäure enthielt. Die Panelisten wurden gebeten, diese Testlösung mit der oben beschriebenen Methode in Bezug auf ihre Bitterkeit und Adstringenz zu bewerten. In einem anschließenden Test wurden beide Deskriptoren (Bitterkeit; Adstringenz) sowohl für die (nicht erfindungsgemäße) Testlösung als auch für eine erfindungsgemäße Lösung zeitabhängig bewertet, die durch Zusatz von 50 ppm (2*S*)-2-[(2*E*,4*E*)-Deca-2,4-dienoylamino]propionsäure (Verbindung 4) zur Testlösung hergestellt wurde (t = 0 Sek. ist dabei der Zeitpunkt unmittelbar nach dem Ausspucken der Lösung). Die Ergebnisse beider Zeit-Intensitäts-Kurven wurden statistisch ausgewertet und miteinander verglichen, um einen bitter-, bzw. adstringenz-maskierenden Effekt der (2*S*)-2-[(2*E*,4*E*)-Deca-2,4-dienoylamino]propionsäure (Verbindung 4) zu bestimmen. Die Ergebnisse wurden jeweils dreifach bestimmt und die Mittelwerte der Bitter- und Adstringenz-Reduktion sind in den Tabellen 1 und 2 dargestellt. Für die Signifikanzberechung wurde der Student t-Test, doppelt mit gepaarten Stichproben, herangezogen.

**Tabelle 1: Vergleich: Bitterkeit von Testlösung (ohne (4)) und erfindungsgemäßer Lösung (mit (4)); Reduktion der Bitterkeit; Mittelwert aus 3 Vergleichstests**

| Zeit (Sek.) | Bitterkeit von Testlösung ohne (4) | Bitterkeit von erfindungsgemäßer Lösung mit (4) | Reduktion (%) |
|---|---|---|---|
| 0 | 5,46 | 5,20 | 5 |
| 10 | 4,65 | 4,21 | 9 |
| 30 | 3,45 | 3,19 | 7 |
| 50 | 2,59 | 2,34 | 10 |
| 70 | 1,96 | 1,56 | 20* |

| | | | |
|---|---|---|---|
| *signifikant (p < 0.1) | | | |

**Tabelle 2: Vergleich: Adstringenz von Testlösung (ohne (4)) und erfindungsgemäßer Lösung (mit (4)); Reduktion der Adstringenz; Mittelwert aus 3 Vergleichstests**

| Zeit (Sek.) | Adstringenz von Testlösung ohne (4) | Adstringenz von erfindungsgemäßer Lösung mit (4) | Reduktion (%) |
|---|---|---|---|
| 0 | 5,67 | 5,29 | 7 |
| 10 | 5,55 | 5,00 | 10* |
| 30 | 4,95 | 3,97 | 20* |
| 50 | 3,81 | 3,14 | 17* |
| 70 | 2,93 | 2,35 | 20* |

| | | | |
|---|---|---|---|
| *signifikant (p < 0.1) | | | |

### Anwendungsbeispiel 3: Flüssige Aromakompositionen

| | Zubereitung (Angaben in Gew.-%) | | | | | | | |
|---|---|---|---|---|---|---|---|---|
| Inhaltsstoff | A | B | C | D | E | F | G | H |
| Verbindung (1) | 1,00 | - | 1,00 | - | 1,00 | - | 0,25 | 0,80 |
| Verbindung (3) | - | 0,60 | - | 0,55 | 0,95 | 0,50 | - | 0,80 |
| *trans*-Pellitorin, 10 %ige Lösung in Propylenglycol und Diethylmalonat 1:1 (m/m) | - | - | - | - | - | 0,50 | - | - |
| Hesperetin | 2,00 | - | - | 2,00 | - | - | - | - |
| Symrise Zucker-Aroma in Propylenglycol-1,2 | 1,50 | 4,00 | - | - | 2,00 | - | 0,30 | - |
| Symrise Milch-Aroma in Propylenglycol-1,2 | - | - | - | 4 | - | - | - | - |
| Symrise Sahne-Aroma in Propylenglycol-1,2 | - | - | 3,00 | 0,30 | 0,50 | 5,00 | 1,00 | 2,50 |
| Propylenglycol | ad 100 | - | ad 100 | ad 100 | ad 100 | ad 100 | - | ad 100 |
| Ethanol | - | ad 100 | - | - | - | - | ad 100 | - |

Die Inhaltsstoffe, ausgenommen Propylenglycol bzw. Ethanol, wurden in den oben angegebenen Mengenverhältnissen gemischt. Anschließend wurde Propylenglycol oder Ethanol zugegeben und sämtliche Inhaltsstoffe wurden durch leichtes Erwärmen vollständig gelöst und homogen gemischt.

### Anwendungsbeispiel 4: Trockene Aromakompositionen

| | Zubereitung (Angaben in Gew.-%) | | | | | | | |
|---|---|---|---|---|---|---|---|---|
| Inhaltsstoff | A | B | C | D | E | F | G | H |
| Verbindung (1) | 25 | - | 0,25 | 0,50 | - | 0,25 | 0,60 | 2,00 |
| Verbindung (3) | - | 15 | - | 0,50 | 0,50 | - | 0,60 | 3,00 |
| *trans*-Pellitorin, 10 %ige Lösung in Propylenglycol und Diethylmalonat 1:1 (m/m) | - | - | 0,25 | - | 0,50 | - | - | 1,00 |
| Zubereitung, umfassend ca. 10 Gew.-% Homoeriodictiol, sprühgetrocknet | - | - | 35,00 | 50,00 | 45,00 | 15,00 | 13,00 | 50,00 |
| Zubereitung, umfassend ca. 10 Gew.-% Hesperetin, sprühgetrocknet | - | - | - | - | 10,00 | 10,00 | 6,00 | - |
| Symrise Abrundungsaroma für Tee, sprühgetrocknet | - | - | 1,00 | - | 30,00 | 5,00 | - | - |
| Symrise Zucker-Aroma in Triacetin | 8,00 | 5,00 | 0,50 | - | - | - | - | - |
| Symrise Vanille, sprühgetrocknet | - | - | - | - | - | - | 3,00 | 10,00 |
| Dextrose wasserfrei | - | ad 100 | - | - | - | ad 100 | - | - |
| Maltodextrin | ad 100 | - | ad 100 | ad 100 | ad 100 | - | ad 100 | ad 100 |

Die Inhaltsstoffe wurden zunächst in Abwesenheit der festen Trägerstoffe (Dextrose bzw. Maltodextrin) in den oben angegebenen Mengenverhältnissen gemischt und anschließend mit Dextrose oder Maltodextrin homogen vermischt.

### Anwendungsbeispiel 5: Sprühgetrocknete Aromakompositionen als Halbfertigwaren zur Aromatisierung von Fertigwaren

| | Zubereitung (Angaben in Gew.-%) | | | | | |
|---|---|---|---|---|---|---|
| Inhaltsstoff | A | B | C | D | E | F |
| Maltodextrin aus Weizen | 24,30 | 24,30 | 24,30 | 24,30 | 24,30 | 24,30 |
| Gummi Arabicum | 6,10 | 6,10 | 6,10 | 6,10 | 6,10 | 6,10 |
| Verbindung (1) | 1,00 | - | 0,40 | - | 1,00 | 0,50 |
| Verbindung (3) | | 0,80 | - | 0,60 | - | 0,50 |
| Symrise Zucker-Aroma in Triacetin | - | 0,50 | 0,20 | - | 0,10 | - |
| Symrise Sahne-Aroma in Triacetin | - | - | - | 0,05 | 0,05 | - |
| Symrise Milch-Aroma in Triacetin | - | - | - | - | - | 0,10 |
| Trinkwasser | ad 100 | ad 100 | ad 100 | ad 100 | ad 100 | ad 100 |

Das Trinkwasser wurde in einem Behälter vorgelegt und das Maltodextrin und das Gummi Arabicum darin gelöst; es resultiert eine Trägerstofflösung. Anschließend wurden die restlichen Inhaltsstoffe mit einem Mixer (Turrax) in diese Trägerstofflösung emulgiert. Die Temperatur des resultierenden Gemisches sollte 30°C nicht überschreiten. Das Gemisch wurde dann sprühgetrocknet (Solltemperatur Eingang: 185 bis 195°C, Solltemperatur Ausgang: 70 bis 75°C). Analog können auch sprühgetrocknete Zubereitungen mit anderen erfindungsgemäßen Aromakompositionen hergestellt werden.

### Anwendungsbeispiel 6: Süßstoffmischung zur Süßung von Kakao-, Kaffee- oder Tee-haltigen Getränken

### Vergleichszubereitung (A)

Erfindungsgemäße Zubereitungen (B bis D), enthaltend erfindungsgemäße Aromakompositionen

| | Zubereitung (Angaben in Gew.-%) | | | |
|---|---|---|---|---|
| Inhaltsstoff | A | B | C | D |
| Saccharin | 1,50 | 1,50 | 1,50 | 1,50 |
| Aromakomposition gemäß Anwendungsbeispiel 4 B | - | 1,70 | 1,00 | 0,60 |
| Aromakomposition gemäß Anwendungsbeispiel 4 A | - | - | - | 1,00 |
| Sorbitol | ad 100 | ad 100 | ad 100 | ad 100 |

Die Zubereitungen A bis D wurden in einer 1 %igen Dosierung mit frisch gebrühtem, schwarzen Kaffee vermischt. Verglichen mit dem Kaffee, der die Zubereitung A (Vergleich) enthielt, war in den Kaffees, die die Zubereitungen B bis D enthielten die Adstringenz und die langanhaltende Süße vermindert. Die metallischen Geschmackseindrücke waren zudem reduziert. Das Kaffeegetränk, enthaltend Zubereitung D, hatte außerdem einen Sucrose-typischeren Geschmack.

### Anwendungsbeispiel 7: Fettarme, gesüßte Joghurt-Zubereitung

### Vergleichszubereitung (A)

Erfindungsgemäße Zubereitungen (B bis D), enthaltend erfindungsgemäße Aromakompositionen

| | Zubereitung (Angaben in Gew.-%) | | | |
|---|---|---|---|---|
| Inhaltsstoff | A | B | C | D |
| Aromakomposition gemäß Anwendungsbeispiel 3 E | - | 0,03 | - | - |
| Aromakomposition gemäß Anwendungsbeispiel 3 A | - | - | - | 0,08 |
| Aromakomposition gemäß Anwendungsbeispiel 3 H | - | - | 0,03 | - |
| Acesulfam K | 0,01 | 0,01 | 0,01 | 0,01 |
| Aspartam | 0,02 | 0,02 | 0,02 | 0,02 |
| Joghurt natur, 0,1% Fett | ad 100 | ad 100 | ad 100 | ad 100 |

Die Aromakompositionen sowie die Süßstoffe wurden mit dem neutralen 0,1 % fetthaltigen Joghurt verrührt. Die resultierenden Zubereitungen wurden anschließend für 3 Tage so gelagert, dass sie reifen konnten.

Verglichen mit Zubereitung A (Vergleich) war in den Zubereitungen B bis D die Adstringenz und die langanhaltend Süße vermindert. Metallische Geschmackseindrücke wurden ebenfalls reduziert. In Zubereitung C wurde eine deutlich stärkere Mundfülle erreicht (nach Sucrose schmeckend).

Analog zu diesem Anwendungsbeispiel können auch Zubereitungen auf Basis anderer fettreduzierter, fettarmer bzw. fettfreier Milchprodukte (z.B. Eiscreme) hergestellt werden.

### Anwendungsbeispiel 8: Sojamilch-Getränk

### Vergleichszubereitung (A)

Erfindungsgemäße Zubereitungen (B bis H), enthaltend erfindungsgemäße Aromakompositionen.

| | Zubereitung (Angaben in Gew.-%) | | | | | | | |
|---|---|---|---|---|---|---|---|---|
| Inhaltsstoff | A | B | C | D | E | F | G | H |
| Aromakomposition gemäß Anwendungsbeispiel 3 G | - | - | 0,10 | - | - | - | - | - |
| Aromakomposition gemäß Anwendungsbeispiel 3 F | - | 0,04 | - | - | - | - | - | - |
| Aromakomposition gemäß Anwendungsbeispiel 3 C | - | - | - | 0,01 | - | - | 0,005 | - |
| Aromakomposition gemäß Anwendungsbeispiel 3 A | - | - | - | - | - | 0,10 | - | - |
| Aromakomposition gemäß Anwendungsbeispiel 3 D | - | - | - | - | 0,15 | - | 0,10 | - |
| Aromakomposition gemäß Anwendungsbeispiel 3 E | - | - | - | - | - | - | - | 0,015 |
| Sojamilch, ohne Zusätze (lokaler Supermarkt) | ad 100 | ad 100 | ad 100 | ad 100 | ad 100 | ad 100 | ad 100 | ad 100 |

Die Aromakompositionen wurden mit der Sojamilch gemischt. Die resultierenden Zubereitungen wurden anschließend für 5 bis 6 Tage so gelagert, dass sie reifen konnten.

Verglichen mit Zubereitung A (Vergleich) war in den Zubereitungen B bis H die Adstringenz vermindert. In den Zubereitungen B bis D, sowie G und H war die Mundfülle deutlich verbessert, der Soja-typische Geschmackseindruck war hingegen reduziert. In den Zubereitungen E und F war neben der Adstringenz besonders die Bitterkeit reduziert.

### Anwendungsbeispiel 9: Soja-Getränk in Kombination mit gamma-Aminobuttersäure

| | Zubereitung (Angaben in Gew.-%) |
|---|---|
| **Inhaltsstoff** | |
| Sojamilch, ohne Zusätze (lokaler Supermarkt) | 99,76 |
| Aromakomposition gemäß Anwendungsbeispiel 3 F | 0,04 |
| 1 % gamma-Aminobuttersäure in Wasser | 0,20 |

Gamma-Aminobuttersäure wurde zunächst in Wasser gelöst und anschließend zusammen mit der Aromakomposition zu einer Sojamilch aus einem lokalen Supermarkt hinzugefügt.

### Anwendungsbeispiel 10: Soja-Frucht-Getränk

### Vergleichszubereitung (A)

Erfindungsgemäße Zubereitungen (B bis D), enthaltend erfindungsgemäße Aromakompositionen

| | Zubereitung (Angaben in Gew.-%) | | | |
|---|---|---|---|---|
| Inhaltsstoff | A | B | C | D |
| Aromakomposition gemäß Anwendungsbeispiel 3 D | - | 0,12 | 0,10 | - |
| Aromakomposition gemäß Anwendungsbeispiel 3A | - | - | - | 0,08 |
| Aromakomposition gemäß Anwendungsbeispiel 3C | - | - | 0,003 | - |
| Süßstoffmix | 0,03 | 0,03 | 0,03 | 0,03 |
| Zucker | 5,00 | 5,00 | 5,00 | 5,00 |
| Fruchtsaftmix aus Fruchtsaftkonzentraten | 50,00 | 50,00 | 50,00 | 50,00 |
| Sojapulver | 5,00 | 5,00 | 5,00 | 5,00 |
| Wasser | ad 100 | ad 100 | ad 100 | ad 100 |

Die Aromakompositionen wurden mit den restlichen Inhaltsstoffen gemischt. Diese Mischungen wurden homogenisiert und anschließend pasteurisiert (15 Min. bei 80 bis 85°C).

Verglichen mit Zubereitung A (Vergleich) waren in den Zubereitungen B bis D die Adstringenz und der Soja-typische Geschmackseindruck vermindert. In Zubereitung B war außerdem die Bitterkeit deutlich reduziert. Sämtliche erfindungsgemäße Zubereitungen (B bis D) wiesen einen deutlich fruchtigeren Geschmack auf als Zubereitung A.

### Anwendungsbeispiel 11: Sojaeiscreme

### Vergleichszubereitung (A)

Erfindungsgemäße Zubereitungen (B bis D), enthaltend erfindungsgemäße Aromakompositionen

| | Zubereitung (Angaben in Gew.-%) | | | |
|---|---|---|---|---|
| Inhaltsstoff | A | B | C | D |
| Aromakomposition gemäß Anwendungsbeispiel 3 F | - | 0,06 | - | - |
| Aromakomposition gemäß Anwendungsbeispiel 3 G | - | - | - | 0,12 |
| Aromakomposition gemäß Anwendungsbeispiel 3 C | - | - | 0,014 | - |
| Sojaeiscreme-Mix (umfassend 12% Saccharose, 8% Glucofructose Sirup, 3% Sojapulver, 4,5% Fett) | ad 100 | ad 100 | ad 100 | ad 100 |

Die Aromakompositionen wurden mit dem Eiscreme-Mix gemischt. Anschließend wurde die Masse im Freezer mit einem Stickstoff-Aufschlag von 100 % gefroren und bei -25°C gelagert.

Verglichen mit Zubereitung A (Vergleich) war in den Zubereitungen B bis D der Soja-typische Geschmackseindruck deutlich reduziert.

### Anwendungsbeisgiel 12: Fettfreier Joghurt

### Vergleichszubereitung (A)

Erfindungsgemäße Zubereitungen (B bis D), enthaltend erfindungsgemäße Aromakompositionen

| | Zubereitung (Angaben in Gew.-%) | | | |
|---|---|---|---|---|
| Inhaltsstoff | A | B | C | D |
| Aromakomposition gemäß Anwendungsbeispiel 3 F | - | 0,03 | - | - |
| Aromakomposition gemäß Anwendungsbeispiel 3 G | - | - | - | 0,10 |
| Aromakomposition gemäß Anwendungsbeispiel 5 F | - | - | 0,015 | - |
| Sucrose | 5,00 | 5,00 | 5,00 | 5,00 |
| Joghurt natur, 0,1% Fett | ad 100 | ad 100 | ad 100 | ad 100 |

Die Aromakompositionen sowie die Sucrose wurden mit dem Joghurt verrührt. Diese Zubereitungen wurden anschließend für 3 Tage so gelagert, dass sie reifen konnten.

Verglichen mit Zubereitung A (Vergleich) war in den Zubereitungen B bis D die Adstringenz vermindert. Zudem wurde die Säure des Joghurts vermindert. In Zubereitung D wurde außerdem eine deutlich stärkere Mundfülle erreicht.

### Anwendungsbeispiel 13: Fettreduzierte Mayonnaise

### Vergleichszubereitung (A)

Erfindungsgemäße Zubereitung (B), enthaltend eine erfindungsgemäße Aromakomposition

| | Zubereitung (Angaben in Gew.-%) | |
|---|---|---|
| **Inhaltsstoff** | A | B |
| Fettreduzierte Mayonnaise | 100 | 99,93 |
| Aromakomposition gemäß Anwendungsbeispiel 3 F | - | 0,07 |

Im Vergleich mit der unbehandelten, fettreduzierten Mayonnaise ohne Aromakomposition (Vergleichszubereitung) war die Adstringenz in Zubereitung B reduziert. Außerdem erhielt die erfindungsgemäße Zubereitung ein besseres Mundgefühl/Fettigkeit.

### Anwendungsbeispiel 14: Grüntee-Zubereitungen

### Vergleichszubereitungen: (A) und (D)

Erfindungsgemäße Zubereitungen (B, C, E und F), enthaltend eine erfindungsgemäße Aromakomposition

| **Inhaltsstoff** | Zubereitung (Angaben in Gew.-%) | | | | | |
|---|---|---|---|---|---|---|
| | **A** | **B** | **C** | **D** | **E** | **F** |
| Grüntee-Extrakt, ca. 16% Catechingehalt | 0,25 | 0,25 | 0,25 | - | - | - |
| Grüntee-Extrakt, ca. 80 % Catechingehalt | | | | 0,20 | 0,20 | 0,20 |
| Aromakomposition gemäß Anwendungsbeispiel 4 D | | 0,10 | - | - | 0,20 | - |
| Aromakomposition gemäß Anwendungsbeispiel 4 E | - | - | 0,10 | - | - | 0,20 |
| Süßstoffmischung (Aspartam, Sucralose, 1:1) | 0,01 | 0,01 | 0,01 | - | - | - |
| Äpfelsäure, Citronensäure | 0,10 | 0,10 | 0,10 | - | - | - |
| Demineralisiertes Wasser | ad 100 | ad 100 | ad 100 | ad 100 | ad 100 | ad 100 |

Der Grünteextrakt, ggfs. die Aromakompositionen, sowie gegebenenfalls Säuren und Süßstoffmischung wurden in 80°C heißem Wasser gelöst und anschließend in Flaschen abgefüllt.

Im Vergleich mit Zubereitung A (Vergleichszubereitung) war in den Zubereitungen B und C die Adstringenz reduziert. Ebenso war im Vergleich mit Zubereitung D (Vergleichszubereitung) in den Zubereitungen E und F die Adstringenz reduziert.

### Anwendungsbeispiel 15: Bitterschokolade

### Vergleichszubereitung (A)

Erfindungsgemäße Zubereitung (B), enthaltend eine erfindungsgemäße Aromakomposition

| | Zubereitung (Angaben in Gew.-%) | |
|---|---|---|
| **Inhaltsstoff** | A | B |
| Bitterschokolade, min. 85 % Kakao (Handelsprodukt) | 100 | 99,7 |
| Aromakomposition gemäß Anwendungsbeispiel 4 G | - | 0,3 |

Die Aromakomposition wurde in die bei 40°C geschmolzene Bitterschokolade eingearbeitet und die resultierende flüssige Masse in Tafelform gegossen und mittels dem Fachmann bekannten Temperungsverfahren abgekühlt, wobei man eine Essschokolade erhielt.

Die so zubereitete Schokolade (Zubereitung B) wurde im Vergleich mit der unbehandelten Bitterschokolade (Vergleichszubereitung) von trainierten Fachleuten als weniger bitter, weniger adstringierend und insgesamt als mehr""abgerundet" bezeichnet.

## Patentansprüche

1. Verbindung der Formel (I) 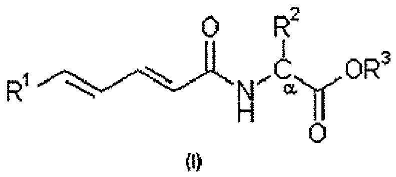 wobei
R¹ eine Alkylgruppe mit 1 bis 6 Kohlenstoffatomen bedeutet,
und
R² Wasserstoff oder eine organische Gruppe mit 1 bis 10 Kohlenstoffatomen bedeutet
und
R³ Wasserstoff oder eine Alkylgruppe mit 1 bis 4 Kohlenstoffatomen bedeutet, sowie
die pharmazeutisch akzeptablen Salze von Verbindungen der Formel (I), in denen R² eine Hydroxy- oder Carboxylgruppe umfasst und/oder R³ Wasserstoff ist.

2. Verbindung oder Salz nach Anspruch 1, wobei R² Wasserstoff bedeutet oder eine organische Gruppe mit 1 bis 10 Kohlenstoffatomen bedeutet, die ausgewählt ist aus der Gruppe bestehend aus verzweigtes Alkyl, unverzweigtes Alkyl, Carboxyalkyl, Alkoxycarbonyl, Carbamoylalkyl, Aminoalkyl, Sulfanyfalkyl, Alkylsulfanylalkyl, Hydroxyalkyl, Guanidinylalkyl, Heterozyklylalkyl, unsubstituiertes Arylalkyl und Hydroxyarylalkyl,

3. Verbindung oder Salz nach einem der vorangehenden Ansprüche,
wobei
R¹ ausgewählt ist aus der Gruppe bestehend aus Methyl, Ethyl, 1-Propyl, 1-Butyl, 1-Pentyl und 1-Hexyl
und/oder
R² ausgewählt ist aus der Gruppe bestehend aus H, -CH₃, -CH₂-CH₃, -CH(CH₃)₂, -CH₂-CH(CH₃)₂, -CH(CH₃)-CH₂-CH₃, -CH₂-CH₂-COOCH₃, -CH₂-C-H₂-COOH, -CH₂-COOCH₃ und -CH₂-COOH
und/oder
R³ ausgewählt ist aus der Gruppe bestehend aus Wasserstoff, Methyl und Ethyl.

4. Verbindung nach einem der vorangehenden Ansprüche, ausgewählt aus der Gruppe bestehend aus:
[(2*E*,4*E*)-Deca-2,4-dienoylamino]essigsäuremethylester (Verbindung 1)
[(2*E*,4*E*)-Deca-2,4-dienoylamino]essigsäure (Verbindung 2)
(2*S*)-2-[(2*E*,4*E*)-Deca-2,4-dienoylamino]propionsäuremethylester (Verbindung 3)
(2*S*)-2-[(2*E*,4*E*)-Deca-2,4-dienoylamino]propionsäure (Verbindung 4) (2*S*)-2-[(2*E*,4*E*)-Deca-2,4-dienoylamino]pentandicarbonsäuredimethylester (Verbindung 5)
und
(2*S*)-2-[(2*E*,4*E*)-Deca-2,4-dienoylamino]pentandicarbonsäure (Verbindung 6)

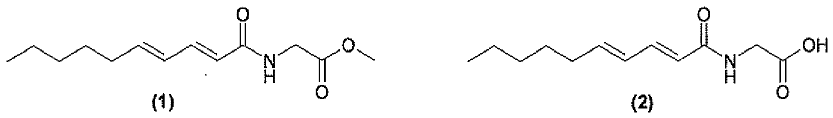

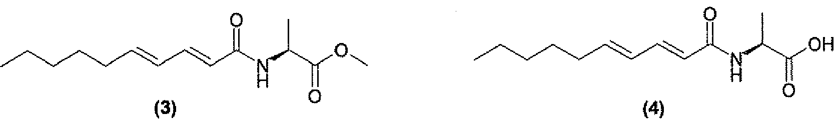

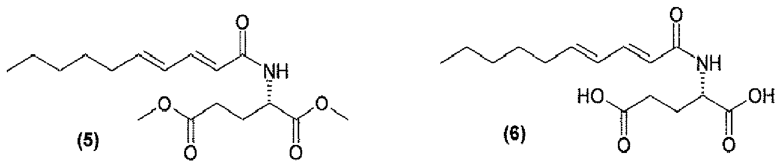

5. Verbindung oder Salz nach einem der vorangehenden Ansprüche, wobei in der Formel (I) R² eine organische Gruppe mit 1 bis 10 Kohlenstoffatomen bedeutet, das alpha-C-Atom chiral ist und die Konfiguration am alpha-C-Atom derjenigen der korrespondierenden L-alpha-Aminosäure H₂N-CR²H-COOH entspricht.

6. Pharmazeutisch akzeptables Salz einer Verbindung der Formel (I) nach einem der vorangehenden Ansprüche, wobei R² eine Hydroxy- oder Carboxylgruppe umfasst und/oder R³ Wasserstoff ist, wobei
das Salz ausgewählt ist aus der Gruppe bestehend aus Natrium-, Kalium-, Ammonium-, Magnesium-, Calcium- und Zinksalz.

7. Verwendung
- einer einzelnen Verbindung oder eines einzelnen Salzes nach einem der Ansprüche 1 bis 6
und/oder
- einer Mischung umfassend oder bestehend aus zwei oder mehr unterschiedlichen Verbindungen oder Salzen gemäß einem der Ansprüche 1 bis 6,
zum Maskieren oder Vermindern eines unangenehmen Geschmackseindrucks eines unangenehm schmeckenden Stoffes.

8. Verwendung gemäß Anspruch 7, wobei der unangenehme Geschmackseindruck ausgewählt ist aus der Gruppe bestehend aus adstringierend, bitter, trocken, staubig, mehlig, kalkig und metallisch.

9. Verwendung nach einem der Ansprüche 7 oder 8, in einer zur oralen Aufnahme bestimmten pharmazeutischen oder einer der Ernährung, der Mundpflege oder dem Genuss dienenden Zubereitung.

10. Zubereitung umfassend
a)
- eine Verbindung oder ein Salz nach einem der Ansprüche 1 bis 6
und/oder
- eine Mischung umfassend oder bestehend aus zwei oder mehr unterschiedlichen Verbindungen oder Salzen nach einem der Ansprüche 1 bis 6 und zusätzlich
b)
- ein oder mehrere unangenehm schmeckende Stoffe,
wobei
- die Menge des bzw. der unangenehm schmeckenden Stoffe ausreicht, um in einer Vergleichszubereitung, die keine Verbindung und kein Salz nach einem der Ansprüche 1 bis 6 umfasst, aber ansonsten identisch zusammengesetzt ist, als unangenehmer Geschmack wahrgenommen zu werden,
und wobei
- die Menge der Verbindung, des Salzes oder der Mischung in der Zubereitung ausreicht, um im Vergleich mit der Vergleichszubereitung den unangenehmen Geschmackseindruck des unangenehm schmeckenden Stoffes oder Stoffgemisches sensorisch zu maskieren oder zu vermindern.

11. Zubereitung nach Anspruch 10, wobei der sensorisch maskierte oder verminderte unangenehme Geschmackseindruck des unangenehm schmeckenden Stoffes oder Stoffgemisches einen, zwei, drei, vier, fünf, sechs oder sämtliche der Geschmackseindrücke umfasst ausgewählt aus der Gruppe bestehend aus adstringierend, bitter, trocken, staubig, mehlig, kalkig und metallisch.

12. Zubereitung nach einem der Ansprüche 10 oder 11, wobei der oder die unangenehm schmeckenden Stoffe ausgewählt sind aus der Gruppe bestehend aus: Xanthinalkaloide, Xanthine, Alkaloide, phenolische Glycoside, Flavonoidglycoside, Chalcone oder Chalconglycoside, hydrolysierbare Tannine, nicht-hydrolysierbare Tannine, Flavone, Phenole, Polyphenole, terpenoide Bitter- und Gerbstoffe, Absinthin aus Wermut, Amarogentin aus Enzian, metallische Salze, pharmazeutische Wirkstoffe, Vitamine, Denatoniumbenzoat, Sucraloseoctaacetat, Eisensalze, Aluminiumsalze, Zinksalze, Harnstoff, ungesättigte Fettsäuren, Aminosäuren, Peptide, Proteine, Saponine und Isoflavonoide

13. Zubereitung nach einem der Ansprüche 10 bis 12, zudem umfassend
- einen oder mehrere Aromastoffe, die einen milchig-sahnigen und Mundfülle gebenden und/oder einen süss-karamelligen Geschmackseindruck vermitteln
sowie
- ein oder mehrere Lösungsmittel, feste oder flüssige Trägerstoffe, Hilfsstoffe und/oder Stabilisierungsmittel.

14. Zubereitung nach einem der Ansprüche 10 bis 13, ferner umfassend zumindest eine weitere Substanz zum Verändern, Maskieren oder Vermindern des unangenehmen Geschmackseindrucks eines unangenehm schmeckenden Stoffes oder Stoffgemisches.

15. Zubereitung nach einem der Ansprüche 10 bis 14, **dadurch gekennzeichnet, dass** die Zubereitung, bezogen auf ihr Gesamtgewicht,
eine Gesamtmenge an Verbindungen oder Salzen nach einem der Ansprüche 1 bis 6 im Bereich von
0,1 ppm, bevorzugt 1 ppm,
bis
100 ppm, bevorzugt 75 ppm, besonders bevorzugt 50 ppm,
umfasst.
